# EUROPEAN PATENT SPECIFICATION

(11) **EP 4 043 816 B1**
(45) Date of publication and mention of the grant of the patent: **13.11.2024**
(21) Application number: 22152410.1
(22) Date of filing: 20.01.2022
(51) Int. Cl.: F25D 23/02, F25D 27/00

(54) **LIGHTING MODULE AND REFRIGERATOR HAVING DISPENSER**
BELEUCHTUNGSMODUL UND KÜHLSCHRANK MIT EINEM SPENDER
MODULE D'ÉCLAIRAGE ET RÉFRIGÉRATEUR DOTÉ D'UN DISTRIBUTEUR

(30) Priority: 10.02.2021 KR 20210019135; 10.02.2021 KR 20210019151
(43) Date of publication of application: 17.08.2022
(60) Divisional application: 24196954.2
(73) Proprietor: LG Electronics Inc., SEOUL 07336 (KR)
(72) Inventor: LEE, Sunghun, 08592 Seoul (KR); KIM, Hoon, 08592 Seoul (KR); KIM, Minki, 08592 Seoul (KR); PARK, Jihoon, 08592 Seoul (KR)
(74) Representative: Ter Meer Steinmeister & Partner

(56) References cited:
- US-A1- 2012 103 001
- US-A1- 2019 346 198

## Description

### BACKGROUND

The present disclosure relates to a dispenser and a refrigerator provided with the dispenser.

In general, a refrigerator is a home appliance for storing foods in an internal storage space, which is shield by a door, at a low temperature by low temperature air. For this, the refrigerator cools the inside of the storage space using cool air generated by heat-exchanging with a refrigerant that circulates a cooling cycle to store the foods in an optimum state.

In recent years, refrigerators have become increasingly multi-functional with changes of dietary lives and gentrification of products, and refrigerators having various structures and convenience devices for convenience of users and for efficient use of internal spaces have been released.

A dispenser that is capable of being manipulated from the outside may be provided in a front surface of a door of the refrigerator, and water purified by a filter device may be dispensed through the dispenser. In addition, the dispenser may be connected to an ice maker that makes ice and may dispense ice through the manipulation of the dispenser.

In addition, a display for displaying manipulation and operation states of the dispenser may be provided at one side of the dispenser.

As an example, Korean Patent Registration No. 10-1815383 discloses a structure provided with a display assembly including a manipulation portion that is touched by a user and a display that displays an operation state and a set state of a refrigerator.

In addition, a device for sterilizing a tube or tank connected to the dispenser using ultraviolet light may be provided so that clean water is dispensed through the dispenser.

As an example, Korean Patent Publication No. 10-2020-0095996 discloses a dispenser structure that sterilizes the inside of a nozzle tube by providing a light source, which irradiates ultraviolet rays toward the inside of the nozzle tube, at one side of the nozzle tube from which purified water is dispensed from the dispenser. US 2019/346198 A1 relates to a beverage dispenser and a refrigeration appliance, in particular a household refrigeration appliance, in which such a beverage dispenser is installed. US 2012/103001 A1 relates to providing a container location indicator to a user of a dispenser, such as those found in consumer appliances, including refrigeration appliances.

### SUMMARY

It is an object of the present disclosure to provide a dispenser having a simple and compact structure to improve assemblability and productivity, and a refrigerator having the dispenser.

It is an object of the present disclosure to also provide a dispenser having a simple and neat outer appearance structure and a refrigerator having the dispenser.

It is an object of the present disclosure to also provide a dispenser capable of intuitively inducing user's manipulation and displaying a state and a refrigerator having the dispenser.

It is an object of the present disclosure to also provide a dispenser provided in a ring shape and provided with a ring light capable of emitting light with uniform brightness from an outer surface of a dispensing portion and a refrigerator having the dispenser.

It is an object of the present disclosure to also provide a dispenser provided with a display that is provided on a touch assembly, displays a manipulation state, has a simple processing method, and is capable of ensuring sufficient visibility, and a refrigerator having the dispenser.

It is an object of the present disclosure to also provide a dispenser in which a touch assembly is provided into a capacitive manner having excellent touch sensitivity and a manipulation portion maintaining constant touch sensitivity is provided, and a refrigerator having the dispenser.

The invention is defined by the features of independent claim 1. Further embodiments are defined by the appended dependent claims.

In a further embodiment, a lighting assembly includes: a light substrate on which a first LED and a second LED are installed to be spaced apart from each other; a plurality of extension portions made of a light transmissive material, the plurality of extension portions being configured to face the first LED and the second LED, respectively; and a light guide connected to each of the extension portions and provided with a round portion having a curved section so as to transmit and emit light emitted from the LEDs.

In a further embodiment, a lighting assembly includes: a light substrate on which a first LED and a second LED are installed to be spaced apart from each other; a light guide having a round portion with an arc shape and extension portions extending from either end of the round portion to face the LEDs, wherein the round portion surrounds the ice chute and/or the nozzle so as to transmit and emit light emitted from the LEDs. The light guide may be made of a light transmissive material. The lighting assembly may further comprise a diffusion member provided outside the light guide to be exposed to an outside of the light assembly so that the light emitted from the light guide is diffused.

In another embodiment, a dispenser device includes: a dispenser case; a touch case installed in the dispenser case and provided with a manipulation portion having a curved section on a front surface thereof; the lighting assembly according to any one of the herein described embodiments; a touch assembly installed behind the touch case so as to be touched by a user; and a nozzle disposed below the touch assembly to discharge water downward, wherein, in the touch assembly, a first touch sensor and a second touch sensor are installed on a touch substrate having a flat plate shape, a first transfer member and a first elastic member are installed between the first touch sensor and the manipulation portion, a second transfer member and a second elastic member are installed between the second touch and the manipulation portion, and an inclination portion is disposed on the second elastic member.

In a further embodiment, a dispenser device includes: a dispenser case; a touch case installed in the dispenser case and provided with a manipulation portion having a curved section on a front surface thereof; a touch assembly installed behind the touch case so as to be touched by a user; and a nozzle disposed below the touch assembly to discharge water downward, wherein, in the touch assembly, a first touch sensor and a second touch sensor are installed on a touch substrate having a flat plate shape, a first transfer member and a first elastic member are installed between the first touch sensor and the manipulation portion, a second transfer member and a second elastic member are installed between the second touch and the manipulation portion, and an inclination portion is disposed on the second elastic member. Optionally, the dispenser device may include the lighting assembly according to any one of the herein described embodiments.

In a further embodiment, a dispenser for a refrigerator includes: an ice chute defining a passage for dispensing ice and/or a nozzle for dispensing water; and a touch assembly for receiving a touch input of a user to operate the dispenser, the touch assembly comprising: a manipulation portion having a curved shape or arc shape, a first touch sensor and a second touch sensor installed on at least one touch substrate that has a flat plate shape, the touch sensors being covered by the manipulation portion, a first elastic member installed between the first touch sensor and the manipulation portion at a center of the manipulation portion, and a second elastic member installed between the second touch sensor and the manipulation portion at a periphery of the manipulation portion.

In a further another embodiment, a refrigerator includes a cabinet defining a storage space; a door configured to open and close the storage space; and a dispenser according to any one of the herein described embodiments, the dispenser being provided on the door, e.g. on a front surface thereof.

In a further embodiment, a refrigerator including a dispenser device includes: a cabinet configured to define a storage space; a door configured to open and close the storage space; a dispenser provided on a front surface of the door to dispense ice; a dispenser case mounted on the door and configured to define a recessed space; an ice chute provided in the recessed space and configured to define a passage through which the ice is dispensed; and the lighting assembly according to any one of the herein described embodiments.

In a further embodiment, a refrigerator includes: a dispenser for dispensing ice and/or water, and a lighting assembly according to any one of the herein described embodiments. The lighting assembly may surround a nozzle of the dispenser for dispensing water and/or an ice chute of the dispenser to dispense ice. The refrigerator may comprise: a cabinet defining a storage space; a door configured to open and close the storage space; a dispenser provided on the door, e.g. on a front surface thereof, and including an ice chute defining a passage for dispensing ice and/or a nozzle for dispensing water; and a lighting assembly mounted to surround the ice chute and/or the nozzle. The lighting assembly may be according to any one of the herein described embodiments.

In a further embodiment, a refrigerator including a dispenser device includes: a cabinet configured to define a storage space; a door configured to open and close the storage space; a dispenser provided on a front surface of the door to dispense ice; a dispenser case mounted on the door and configured to define a recessed space; an ice chute provided in the recessed space and configured to define a passage through which the ice is dispensed; and a lighting assembly mounted to surround the ice chute, wherein the lighting assembly includes: a light substrate on which a first LED and a second LED are installed to be spaced apart from each other; a plurality of extension portions made of a light transmissive material, the plurality of extension portions being configured to face the first LED and the second LED, respectively; and a light guide connected to each of the extension portions and provided with a round portion having a curved section to surround the ice chute so as to transmit and emit light emitted from the LEDs.

The light assembly, the dispenser device and/or the refrigerator according to any one of the above embodiments may include one or more of the following features:
The light assembly may be a ring light assembly.

The light guide may comprise a round portion disposed along a circumference of a dispensing portion body through which a nozzle, from which purified water is discharged, passes, and an extension portion extending from a rear end of the round portion to the LED.

The light assembly may further comprise a pattern portion disposed along an inner circumferential surface of the round portion to reflect incident light to an outside. The pattern portion may have a concave shape, in which a groove is repeated. The groove may have a width that gradually increases from the rear end of the round portion toward a front side of the round portion and an interval that gradually decreases from the rear end of the round portion toward the front side of the round portion.

The light diffusion member comprises a diffusion member base configured to support a bottom surface of the light guide. The light diffusion member may further comprise a front extension portion extending upward from a front end of the diffusion member base, the front extension portion being in contact with a front surface of the light guide. The light diffusion member may further comprise an exposed portion configured to protrude from the front extension portion so as to be exposed to an outside of the dispensing portion body.

The extension portion may be disposed higher than the round portion.

The light guide may comprise a connection portion configured to connect the round portion to the extension portion and extending to be inclined.

A through-hole, through which the connection portion passes, may be defined in a bottom surface of the upper body.

The round portion or the light guide may be disposed on the bottom surface of the upper body.

The manipulation portion of the dispenser device may have a curved or bent shape or an arc shape, e.g. the manipulation portion may have a rounded front surface. The manipulation portion may be convexly curved, i.e. in view of a user.

The first elastic member of the dispenser device may be disposed at a center of a rear surface of the manipulation portion.

The second elastic member of the dispenser device may be disposed to be spaced apart from each of both left and right sides of the first elastic member.

The inclination portion may be inclined closer to the rear surface of the manipulation portion in a direction away from the first elastic member.

The dispenser may include a dispenser case mounted on the door to define a recessed space in which the ice chute and/or the nozzle is provided.

The touch assembly for operating the dispenser may comprise: a manipulation portion having a curved shape or arc shape, a first touch sensor and a second touch sensor installed on at least one touch substrate that has a flat plate shape, the touch sensors being covered by the manipulation portion, a first elastic member installed between the first touch sensor and the manipulation portion at a center of the manipulation portion, and a second elastic member installed between the second touch sensor and the manipulation portion at a periphery of the manipulation portion. The first elastic member and the second elastic member may respectively have a surface in contact with the manipulation portion. Said surface may have a shape formed corresponding to the manipulation portion. In other words, a front surface of the first elastic member and/or of the second elastic member may be in contact with a rear surface of the manipulation portion and formed corresponding to the rear surface of the manipulation member. The rear surface of the manipulation member may have a curved shape or arc shape. The first touch sensor and/or the first elastic element may be installed in a center of the manipulation portion, e.g. in a region of the manipulation portion protruding most forward from the refrigerator. The second touch sensor and/or the second elastic element may be installed in a periphery of the manipulation portion, i.e. in a lateral region of the manipulation portion and/or in a region of the manipulation portion protruding less forward than the center region. For instance, the second touch sensor and/or the second elastic element may be installed left and/or right of the first touch sensor. The touch assembly may further comprise a first transfer member installed between the first touch sensor and the manipulation portion, and a second transfer member installed between the second touch sensor and the manipulation portion. The manipulation portion of the touch assembly may be provided on a portion of a touch case of the touch assembly. The touch substrate, the touch sensors, the transfer members and/or the elastic members may be provided behind the touch case, i.e. may be covered by the touch case. The manipulation portion may have a curved surface, e.g. the manipulation portion may have a rounded front surface, for receiving a touch input of a user. The manipulation portion may have a curved or bent shape or an arc shape. The manipulation portion and/or the front surface thereof and/or the rear surface thereof may be convexly curved, i.e. in view of a user. The front surface of the manipulation portion may denote the surface of the manipulation portion for receiving a touch input by the user, i.e. facing a user. The rear surface of the manipulation portion may denote the surface of the manipulation portion opposite to said front surface. The nozzle and/or the ice chute may be disposed below the touch assembly and/or below the manipulation portion. The touch assembly may further comprise a guide member provided between the manipulation portion and the touch substrate. A front surface of the guide member may have a rounded shape corresponding to the (rear surface of the) manipulation portion. The guide member may have a guide hole accommodating the first elastic member and the second elastic member. The touch assembly may further comprise a display on the manipulation portion and a light emitting member provided on the touch substrate, wherein the guide member is disposed between the display and the light emitting member and configured to guide light emitted from the light emitting member to the display. The guide member may have a light hole extending from the touch substrate to the manipulation portion to guide the light emitted from the light emitting member toward the display. A blocking layer configured to block light may be provided on a surface, e.g. front and/or rear surface, of the manipulation portion. The display may be formed by removing the blocking layer, e.g. by laser processing.

The touch assembly of the dispenser device may further comprise a guide member provided on a front surface of the touch substrate to guide light emitted from a light emitting member provided on the touch substrate. A front surface of the guide member may have a rounded shape corresponding to the rear surface of the manipulation portion. A guide hole configured to define a space, into which the first elastic member and the second elastic member are accommodated, may be defined in the guide member. The first elastic member and the second elastic member may further protrude forward than an opened front surface of the guide hole. The touch assembly of the dispenser device may comprise a light emitting member. The touch assembly of the dispenser device may further comprise a blocking layer configured to block light emitted from the light emitting member so as not to be transmitted on an outer surface of the touch case. The touch assembly of the dispenser device may further comprise a display configured to transmit the light by removing the blocking layer at a position corresponding to the light emitting member. The guide member may have a light hole extending from the front surface of the touch substrate to the rear surface of the manipulation portion to guide the light emitted from the light emitting member toward the display. The display may be configured so that the light is transmitted by removing a portion of the blocking layer through laser processing.

The lighting assembly may comprise a diffusion member provided outside the light guide so as to be exposed between the touch assembly and the lighting assembly so that the light emitted from the light guide is diffused.

According to a further embodiment, a lighting assembly for a dispenser of a refrigerator, comprises: a dispensing body portion including an upper body and a lower body coupled to each other, the dispensing body portion having a cylindrical shape; a light guide disposed between the upper body and the lower body and having a round portion with an arc shape; and at least one LED disposed to face one end of the light guide, i.e. such that light emitted from the LED is allowed to enter the light guide.

According to the invention, a light diffusion member is provided to be disposed between the upper body and the lower body, the light diffusion member having an arc shape surrounding or covering an outer circumferential portion of the light guide, e.g. the round portion of the light guide. In this case, a circumferential outer portion of the light diffusion member is exposed to an outside of the dispensing body portion as an exposed portion to emit light. The light diffusion member may include: a diffusion member base supporting the light guide and a front extension portion protruding from the diffusion member base at an outer circumferential portion thereof, the front extension portion being in contact with the outer circumferential portion of the light guide. The front extension portion may protrude from the diffusion member base towards the upper body. The front extension portion may be covered by the upper body. The exposed portion may protrude from the front extension portion and/or from the diffusion member base so as to be exposed to an outside of the dispensing portion body.

The round portion of the light guide may be disposed along a circumference of a dispensing portion body. The light guide may further comprise an extension portion extending from an end of the round portion towards the LED, i.e. to face the LED. The round portion may be closer to the lower body than the extension portion. That is, the round portion may extend in a different plane than the extension portion. The light guide may further comprise a connection portion connecting the round portion and the extension portion and being inclined, e.g. with respect to the round portion and/or the extension portion.

A pattern portion may be formed on an inner circumferential surface of the round portion to direct light towards the outside of the dispensing portion body. The pattern portion may include a plurality of grooves extending in parallel and/or in axial direction and/or perpendicular to a circumferential direction. Here, axial direction may refer to a direction parallel to an (virtual) axis of the cylindrical dispensing portion body. Likewise, circumferential direction may refer to a direction parallel to a direction along the circumferential surface of the cylindrical dispensing portion body. A width of the grooves may gradually increase from the ends of the arc-shaped round portion toward a center thereof and/or wherein an interval between adjacent grooves may gradually decrease from the ends of the arc-shaped round portion toward the center thereof. The connection portion may pass through a through-hole defined in a bottom surface of the upper body. That is, the connection portion may pass into an inside the upper body. The round portion may be disposed on an outer surface of the upper body. That is, the round portion may be disposed on the bottom surface of the upper body, e.g. outside of the upper body. The LED may be mounted on a light substrate provided in or at the upper body.

The dispensing portion body may include at least one of a nozzle insertion portion to accommodate a nozzle for dispensing water and/or a lower opening to allow an ice chute to pass through.

The light guide may be made of a light transmissive material.

According to a further embodiment, a refrigerator is provided including: a dispenser for dispensing ice and/or water, and a lighting assembly according to any one of the herein described embodiments. The lighting assembly may surround a nozzle of the dispenser for dispensing water and/or an ice chute of the dispenser to dispense ice. The refrigerator may comprise: a cabinet defining a storage space; a door configured to open and close the storage space; a dispenser provided on the door, e.g. on a front surface thereof, and including an ice chute defining a passage for dispensing ice and/or a nozzle for dispensing water; and a lighting assembly mounted to surround the ice chute and/or the nozzle. The lighting assembly may be according to any one of the herein described embodiments. The lighting assembly may comprise: a light substrate on which a first LED and a second LED are installed to be spaced apart from each other; a light guide having a round portion with an arc shape and extension portions extending from either end of the round portion to face the LEDs, wherein the round portion surrounds the ice chute and/or the nozzle so as to transmit and emit light emitted from the LEDs. The light guide may be made of a light transmissive material. The lighting assembly may further comprise a diffusion member provided outside the light guide to be exposed to an outside of the light assembly so that the light emitted from the light guide is diffused.

The dispenser may include a dispenser case mounted on the door to define a recessed space in which the ice chute and/or the nozzle is provided.

The refrigerator may include a touch assembly for receiving a touch input of a user to operate the dispenser, the touch assembly comprising: a manipulation portion having a curved shape or arc shape, a first touch sensor and a second touch sensor installed on at least one touch substrate that has a flat plate shape, the touch sensors being covered by the manipulation portion, a first elastic member installed between the first touch sensor and the manipulation portion at a center of the manipulation portion, and a second elastic member installed between the second touch sensor and the manipulation portion at a periphery of the manipulation portion. The first elastic member and the second elastic member may respectively have a surface in contact with the manipulation portion. Said surface may have a shape formed corresponding to the manipulation portion. In other words, a front surface of the first elastic member and/or of the second elastic member may be in contact with a rear surface of the manipulation portion and formed corresponding to the rear surface of the manipulation member. The rear surface of the manipulation member may have a curved shape or arc shape.

The first touch sensor and/or the first elastic element may be installed in a center of the manipulation portion, e.g. in a region of the manipulation portion protruding most forward from the refrigerator. The second touch sensor and/or the second elastic element may be installed in a periphery of the manipulation portion, i.e. in a lateral region of the manipulation portion and/or in a region of the manipulation portion protruding less forward than the center region. For instance, the second touch sensor and/or the second elastic element may be installed left and/or right of the first touch sensor.

The touch assembly may further comprise a first transfer member installed between the first touch sensor and the manipulation portion, and a second transfer member installed between the second touch sensor and the manipulation portion.

The manipulation portion of the touch assembly may be provided on a portion of a touch case of the touch assembly. The touch substrate, the touch sensors, the transfer members and/or the elastic members may be provided behind the touch case, i.e. may be covered by the touch case.

The manipulation portion may have a curved surface, e.g. the manipulation portion may have a rounded front surface, for receiving a touch input of a user. The manipulation portion may have a curved or bent shape or an arc shape. The manipulation portion and/or the front surface thereof and/or the rear surface thereof may be convexly curved, i.e. in view of a user. The front surface of the manipulation portion may denote the surface of the manipulation portion for receiving a touch input by the user, i.e. facing a user. The rear surface of the manipulation portion may denote the surface of the manipulation portion opposite to said front surface.

The nozzle and/or the ice chute may be disposed below the touch assembly and/or below the manipulation portion.

The touch assembly may further comprise a guide member provided between the manipulation portion and the touch substrate. A front surface of the guide member may have a rounded shape corresponding to the (rear surface of the) manipulation portion.

The guide member may have a guide hole accommodating the first elastic member and the second elastic member.

The touch assembly may further comprise a display on the manipulation portion and a light emitting member provided on the touch substrate, wherein the guide member is disposed between the display and the light emitting member and configured to guide light emitted from the light emitting member to the display. The guide member may have a light hole extending from the touch substrate to the manipulation portion to guide the light emitted from the light emitting member toward the display. A blocking layer configured to block light may be provided on a surface, e.g. front and/or rear surface, of the manipulation portion. The display may be formed by removing the blocking layer, e.g. by laser processing.

The details of one or more embodiments are set forth in the accompanying drawings and the description below. Other features will be apparent from the description and drawings, and from the claims.

### BRIEF DESCRIPTION OF THE DRAWINGS

Fig. 1 is a front view of a refrigerator according to an embodiment.
Fig. 2 is a perspective view of a dispenser mounted in a door of the refrigerator.
Fig. 3 is a partial enlarged view illustrating a dispensing portion of the dispenser.
Fig. 4 is an exploded perspective view of the dispenser.
Fig. 5 is a rear perspective view of a touch assembly that is one component of the dispenser.
Fig. 6 is a rear exploded perspective view of the touch assembly that is one component of the dispenser.
Fig. 7 is a front exploded perspective view of the touch assembly.
Fig. 8 is a cross-sectional view taken along line VIII-VIII' of Fig. 3.
Fig. 9 is a cross-sectional view taken along line IX-IX' of Fig. 3.
Fig. 10 is a cross-sectional view taken along line X-X' of Fig. 4.
Fig. 11 is a schematic view illustrating a viewing angle of a user when the user sees the touch assembly at the front.
Fig. 12 is a perspective view illustrating a state in which a lighting assembly, which is one component of the dispenser, the ice chute, and a nozzle assembly are coupled to each other.
Fig. 13 is a cross-sectional view taken along line XIII-XIII' of Fig. 3.
Fig. 14 is an exploded perspective view of the lighting assembly, the ice chute, and the nozzle assembly.
Fig. 15 is a perspective view of the lighting assembly.
Fig. 16 is a front exploded perspective view illustrating a state in which the lighting assembly is disassembled.
Fig. 17 is a bottom exploded perspective view illustrating the state in which the lighting assembly is disassembled.
Fig. 18 is a perspective view of a light guide that is one component of the lighting assembly.
Fig. 19 is a perspective view of a diffusion member that is one component of the lighting assembly.
Fig. 20 is a plan view of an upper body that is one component of the lighting assembly.
Fig. 21 is an enlarged view of a portion A of Fig. 15.
Fig. 22 is a cutaway perspective view taken along line XXII-XXII' of Fig. 15.
Fig. 23 is a cross-sectional view taken along line XXIII-XXIII' of Fig. 2.
Fig. 24 is a view illustrating a state of the dispensing portion when ice is dispensed through the dispenser.
Fig. 25 is a perspective view illustrating an assembled state of the light guide and the diffusion member.
Fig. 26 is a cross-sectional view taken along line XXVI-XXVI' of Fig. 21.
Fig. 27 is a view illustrating a state of the dispensing portion when water is dispensed through the dispenser.
Fig. 28 is a view illustrating a state of the dispensing portion when a nozzle of the dispenser is sterilized.
Fig. 29 is a perspective view of a light guide according to another embodiment.
Fig. 30 is a cross-sectional view illustrating a state in which the light guide is assembled.
Fig. 31 is a front view of a dispenser according to another embodiment.
Fig. 32 is a perspective view illustrating a driving circuit of the dispenser according to another embodiment.

### DETAILED DESCRIPTION OF THE EMBODIMENTS

Hereinafter, detailed embodiments of the present invention will be described in detail with reference to the accompanying drawings.

In addition, the embodiment will be described with an example of a side-by-side type (or double-door type) refrigerator in which a pair of doors are disposed at both left and right sides for convenience of explanation and understanding, and it should be noted in advance that the present disclosure is applicable to all refrigerators provided with a dispenser.

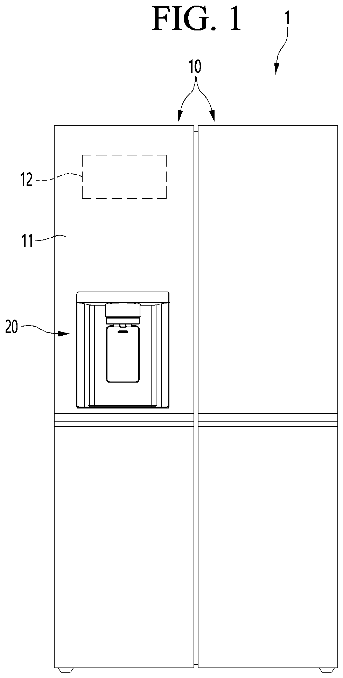

A direction will be defined prior to the explanation. In Fig. 1, a direction in which an outer plate of a door is disposed is referred to as a front direction, a backward direction with respect to the outer plate is referred to as a rear direction, a direction facing a bottom surface on which the refrigerator is installed is referred to as a downward direction, and a direction away from the bottom surface on which the refrigerator is installed is referred to as an upward direction.

Fig. 1 is a front view of a refrigerator according to an embodiment.

Referring to Fig. 1, a refrigerator 1 according to an embodiment includes a cabinet defining a storage space and a door 10 coupled to the cabinet to open and close the storage space. Here, an outer appearance of the refrigerator 1 may be defined by the cabinet and the door 10.

The door 10 may be arranged side by side at both left and right sides and may open and close a space partitioned into both the left and right sides by rotation. For example, the storage space of the cabinet may be divided into both the left and right sides to define a freezing compartment and a refrigerating compartment, and the door 10 may include a freezing compartment door that opens and closes the freezing compartment and a refrigerating compartment door that opens and closes the refrigerating compartment.

The door 10 may define most of an outer appearance of a front surface of the refrigerator 1. To define the outer appearance of the refrigerator 1, a front surface of the door 10 may be defined by an outer plate 11. The outer plate 11 may be made of, for example, a metal material or a material having a metal texture.

In addition, a dispenser 20 may be provided in the door 10. The dispenser 20 may be disposed on the front surface of the door 10 to dispense purified water from the outside in a state in which the door 10 is closed
The dispenser 20 may be provided to have the same or similar color and texture to the outer plate 11 so as to harmonize with the outer plate 11. For example, when the outer plate 11 is made of a metal material, main components defining an outer appearance of the dispenser 20 may be made of a metal material or a plastic material, and a metal texture coating or film may be attached to a surface thereof

An ice maker 12 that makes and stores ice may be provided above the dispenser 20, and the ice maker 12 may be connected to the dispenser 20 to dispense ice through manipulation of the dispenser 20.

The ice maker 12 may be provided inside the freezing compartment or in a rear surface of the freezing compartment door. In addition, when the dispenser 20 is provided in the refrigerating compartment door, the ice maker 12 may be provided in an insulated ice making chamber in a rear surface of the refrigerating compartment door.

The dispenser 20 may be disposed at a position at which the user's manipulation is easy, and the dispenser 20 may be configured so that the user's manipulation is easy, and an operation state is displayed.

Hereinafter, the structure of the dispenser 20 will be described in more detail with reference to the drawings.

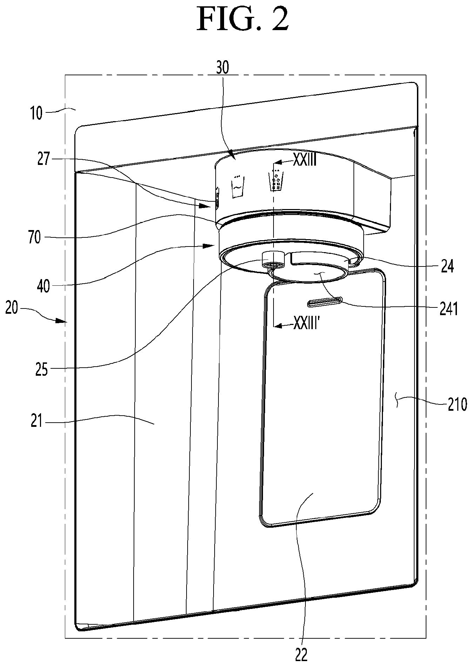

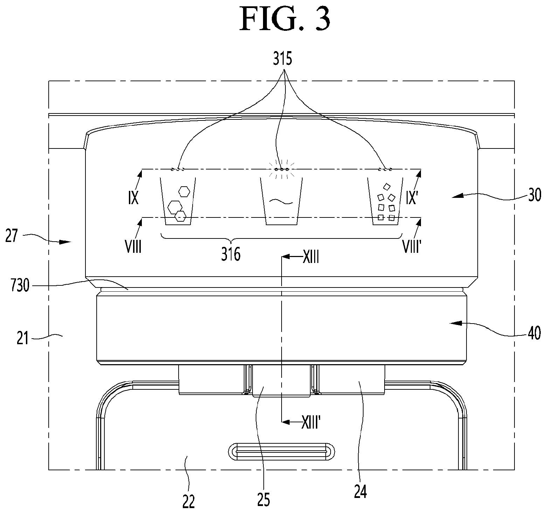

Fig. 2 is a perspective view of the dispenser mounted in the door of the refrigerator. Fig. 3 is a partial enlarged view illustrating the dispensing portion of the dispenser.

As illustrated in the drawings, the dispenser 20 may be mounted to be exposed to the front surface of the door 10 and may be configured to dispense water or ice through manipulation in an inner recessed space.

In detail, the dispenser 20 may be mounted so that a dispenser case 21 defining the recessed space 210 shields an opening of the outer plate 11. A space through which the user accesses a cup or container may be defined by the dispenser case 21, and a space from which water or ice is dispensed may be provided.

In addition, a lever 22 may be provided at a center of the inner surface of the recessed space 210. The dispensing of the water or ice through the dispenser 20 may start by an operation of the lever 22. An upper end of the lever 22 may rotate about a lower end as an axis, and the lever 22 may be rotatably mounted to the dispenser case 21.

An outlet 241 of an ice chute 24 from which ice is discharged may be disposed vertically above the lever 22, and a water outlet 25 through which the purified water is discharged may be disposed at a front end of the ice chute 24. That is, centers of the lever 22, the ice chute 24, and the water outlet 25 may be disposed on the same vertical plane. Thus, when the lever 22 operates using the cup or container, the ice or water discharged through the ice chute 24 and the water outlet 25 may naturally flow toward the cup or container.

In addition, a touch assembly 30 and a lighting assembly 40 may be disposed vertically above the lever 22. A set state of the dispenser 20 may be displayed on the touch assembly 30, and user's touch manipulation may be inputted into the touch assembly 30.

In detail, the touch assembly 30 may be disposed above the lighting assembly 40 and may be disposed on an upper end of the dispenser 20. In addition, the touch assembly 30 may define a portion of an outer appearance of an upper portion of the dispenser 20. In addition, the ice chute 24 may be disposed inside the touch assembly 30 and may be configured to shield the ice chute 24 from the front.

The touch assembly 30 may be provided in a rounded shape when viewed from the front, and a touch portion 316 for the user's touch manipulation and a display 315 for displaying a manipulation state may be disposed on the rounded front surface.

The touch portion 316 may set a function of the dispenser by the user's touch manipulation and may be provided on a front surface of the touch assembly 30 to confirm the manipulation position by printing or surface processing. The touch portion 316 may be provided as a plurality of portions, and the display 315 may be provided in number corresponding to positions corresponding to the touch portion 316.

For example, the touch portion 316 may be divided into three portions that are continuously arranged in a horizontal direction and may be configured to select each of cube-shaped ice, purified water, and crushed ice. The touch portion 316 may be configured so that the selected object appears in the form of a picture or text so that the user intuitively confirms and manipulates the selected object. In addition, the display 315 may be formed above each of the touch portions 316, and the display 315 corresponding to the touch portion 316 selected by the user may be shined so that the user's operation and set state are visualized.

The lighting assembly 40 may be disposed at a lower end of the touch assembly to define a portion of an outer appearance of an upper portion of the dispenser 20. The lighting assembly 40 may be also provided in a round shape having a curvature corresponding to that of the touch assembly 30, and the lower end of the touch assembly 30 may extend. The touch assembly 30 and the lighting assembly 40 may be provided in a coupled state, and the ice chute 24 and the nozzle assembly 50 may be disposed inside the lighting assembly 40. Thus, the touch assembly 30, the lighting assembly 40, and components provided therein may be referred to as the dispensing portion 27.

In addition, the ice chute 24 and the water outlet 25 may protrude further downward than the lower end of the lighting assembly 40 so that at least a portion thereof is exposed through the recessed space 210.

The lighting assembly 40 may include a ring light 730. The ring light 730 may indicate the operation state of the dispenser 20 and may be configured as a portion of a diffusion member 70 to be described below. In addition, the ring light 730 may be disposed along a circumference of the lighting assembly 40. For example, the lighting assembly 40 may be disposed between the lower end of the touch assembly 30 and the lower end of the lighting assembly 40 and may emit light along an outer circumference of the dispensing portion 27.

The ring light 730 may be provided between the touch assembly 30 and the lighting assembly 40. In addition, only a portion of the ring light 730 may be exposed to the outside, and other components constituting the lighting assembly 40 may be disposed inside the touch assembly 30 and the lighting assembly 40 so as not to be exposed to the outside.

In addition, the ring light 730 may emit light in various colors and may display various operation states of the refrigerator 1 and the dispenser in connection with a light emitting pattern or a change in light emission state.

Hereinafter, a structure of the dispenser 20 will be described in more detail.

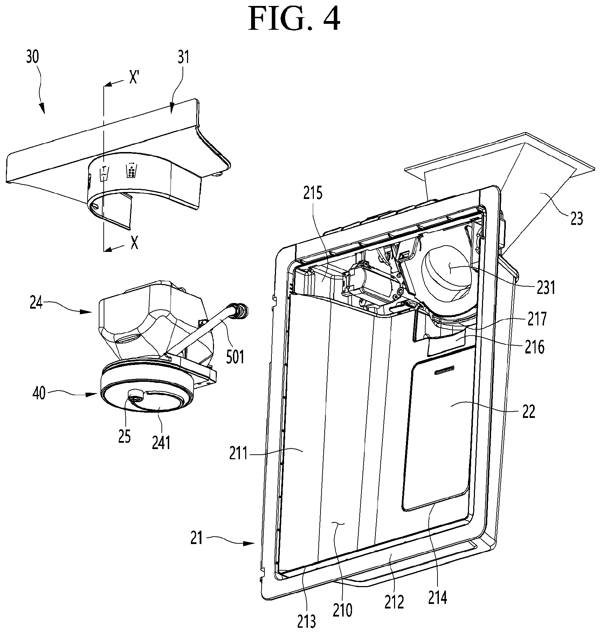

Fig. 4 is an exploded perspective view of the dispenser.

As illustrated in the drawing, the dispenser 20 may include the dispenser case 21, the touch assembly 30, and the lighting assembly 40 as a whole.

The dispenser case 21 may define an overall shape of the dispenser 20, and various components including the touch assembly 30 and the lighting assembly 40 may be mounted therein. In addition, the dispenser case 21 may include a recessed portion 211 defining an inner surface defining the recessed space 210, and a case edge 212 provided along a circumference of the recessed portion 211.

The case edge 212 may be in close contact with a rear surface of the outer plate 11. In addition, a plate insertion groove 213 may be defined between the case edge 212 and the circumference of the recessed portion 211. An end bent along a circumference of an opening of the outer plate 11 may be inserted into the plate insertion groove 213, and the outer plate 11 and the dispenser case 21 may be coupled to each other.

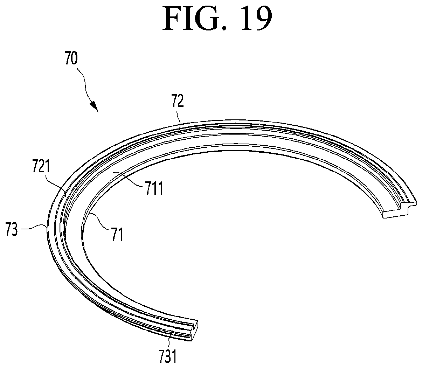

In addition, the recessed portion 211 may provide the recessed space 210. The recessed space 210 may be defined in a shape of which a left and right width become narrower toward the rear side, and a lever mounting portion 214 on which the lever 22 is mounted may be recessed in a rear wall surface of the recessed portion 211. The lever 22 has a lower end that is shaft-coupled at the inside of the lever mounting portion 214 to rotate by pressing an upper end thereof and may be supported at the rear side by a spring 221 (see Fig. 19). Thus, the lever 22 may return to its initial position by elastic force of the spring 221 after the operation. Also, in the non-manipulated state of the lever 22, an upper end of the lever 22 may be disposed at a lower end of a rear surface of the ice chute 24.

In addition, the touch mounting portion 215 to which the touch assembly 30 is mounted may be disposed on both left and right sides of an upper end of the recessed portion 211. The touch mounting portion 215 may be recessed, and an end of the touch case 31 constituting the touch assembly 30 may be inserted into the touch mounting portion 215.

In addition, a chute support 216 may be disposed on the rear surface of the recessed portion 211. The chute support 216 may be disposed above the lever mounting portion 214 and may be recessed so that the rear surface of the ice chute 24 is seated. Thus, the ice chute 24 may be mounted in a state of being stably supported inside the dispensing portion 27 and may be maintained at an accurate mounting position.

In addition, a lighting assembly mounting portion 217 may be disposed above the chute support 216. The lighting assembly mounting portion 217 may be recessed backward so that a lower end of the lighting assembly 40 is inserted. In addition, the lighting assembly mounting portion 217 may be recessed to a size corresponding to a width of a rear end of the lighting assembly 40 to accommodate the rear end of the lighting assembly 40. Thus, the rear end of the lighting assembly 40 may be inserted and mounted in the lighting assembly mounting portion 217. In addition, the lighting assembly mounting portion 217 provides a space in which the lighting module 42 constituting the lighting assembly 40 is accommodated.

A connection passage 23 may be provided in a top surface of the dispenser case 21. The connection passage 23 may provide a passage through which the ice made in the ice maker 12 moves to be dispensed to the dispenser 20 and may extend obliquely backward and upward from the top surface of the ice maker 12. That is, the connection passage 23 may be configured to connect the ice maker 12 to the dispenser 20.

In addition, an opened lower surface of the connection passage 23 may provide an ice outlet 231, and the ice outlet 231 may be disposed above the lighting assembly mounting portion 217, that is, a top surface of the recessed portion 211. The ice outlet 231 may communicate with the ice chute 24. Thus, the ice moving along the connection passage 23 may be discharged through the ice chute 24 from the inside of the recessed space 210.

The top surface of the recessed portion 211 on which the connection passage 23 is provided may be inclined, and a shutter 26 that opens and closes the connection passage 23 may be provided on the top surface of the recessed portion 211. An upper end of the shutter 26 may be shaft-coupled to be rotatably provided and may be opened only when the dispenser 20 operates to dispense the ice so that the ice is discharged through the connection passage 23. In addition, the shutter 26 (see Fig. 19) may be maintained in the closed state except for the operation of discharging the ice to prevent cold air from leaking.

Hereinafter, a structure of the touch assembly 30 will be described in more detail with reference to the accompanying drawings.

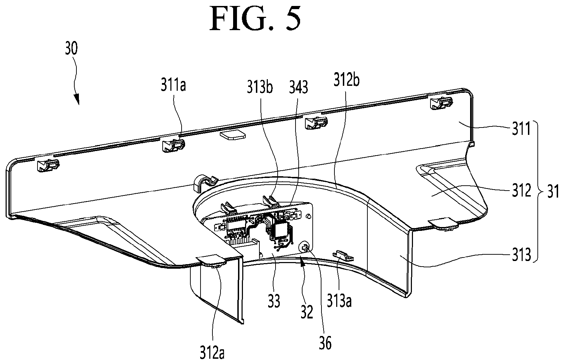

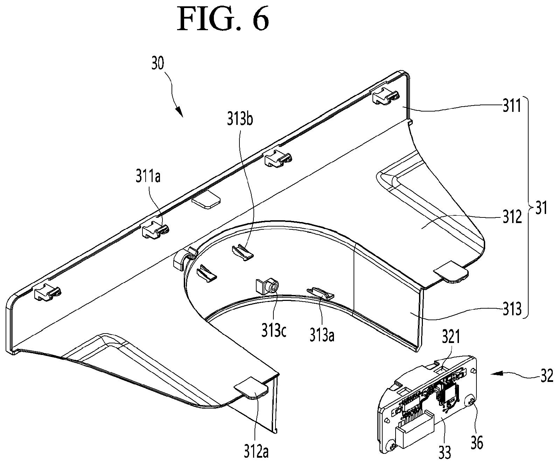

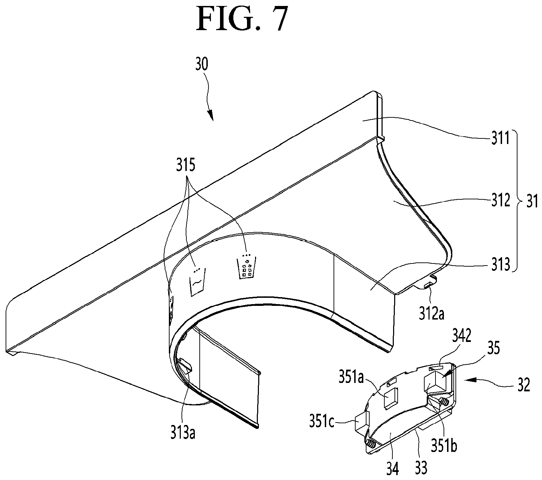

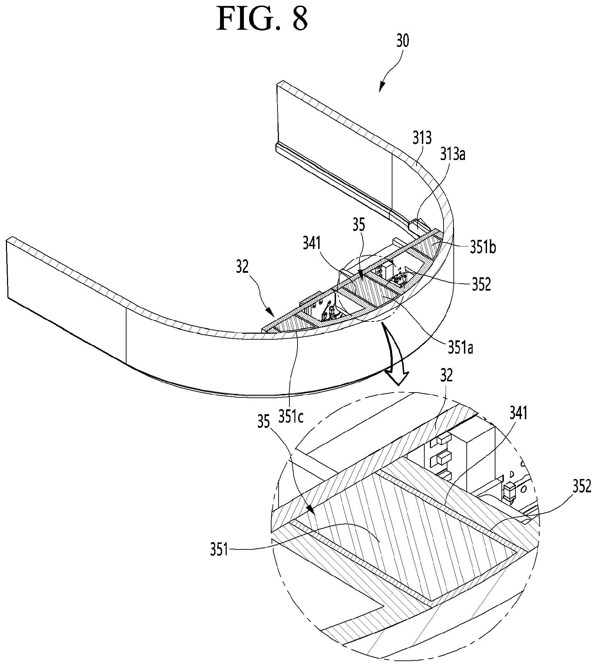

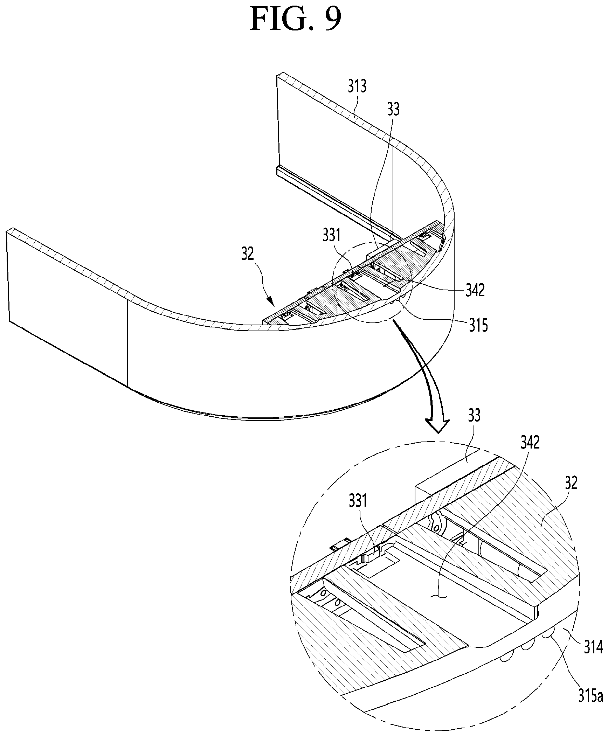

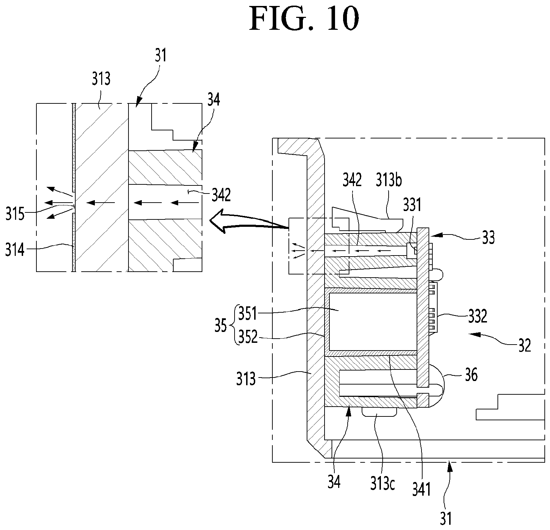

Fig. 5 is a rear perspective view of the touch assembly that is one component of the dispenser. Also, Fig. 6 is a rear exploded perspective view of the touch assembly that is one component of the dispenser. Also, Fig. 7 is a front exploded perspective view of the touch assembly. Also, Fig. 8 is a cross-sectional view taken along line VIII-VIII' of Fig. 3. Also, Fig. 9 is a cross-sectional view taken along line IX-IX' of Fig. 3. Also, Fig. 10 is a cross-sectional view taken along line X-X' of Fig. 4.

As illustrated in the drawings, the touch assembly 30 includes a touch case 31 defining an overall appearance thereof, and a touch device 32 provided inside the touch case 31 to recognize the user's touch operation.

The touch case 31 may be provided to define a portion of an outer appearance of an upper end of the dispenser 20 and the dispensing portion 27 for the user's touch manipulation. The touch case 31 may be injection-molded using a plastic material to implement a complex outer appearance and a mounting structure. Since the touch case 31 defines an upper portion of the dispensing portion 27, the touch case 31 may be referred to as an upper case.

In addition, the touch case 31 may include a front portion 311, a bottom portion 312, and a manipulation portion 313. Of course, if necessary, the touch case 31 may be configured by combining the manipulation portion 313 with other components.

In detail, the front portion 311 may define an upper end of the front surface of the dispenser 20 and may define an upper end of the recessed space 210. In addition, a plurality of front coupling protrusions 311a may be disposed along a circumference of the front portion 311. Each of the front coupling protrusions 311a may be provided in a hook-like shape to be coupled to the dispenser case 21.

The bottom portion 312 extending backward may be disposed at a lower end of the front portion 311. The bottom portion 312 may extend from a lower end of the front portion 311 to the rear surface of the recessed portion 211 and divide the inside of the dispenser case 21 to define the top surface of the recessed space 210. In addition, a bottom portion coupling protrusion 312a extending backward may be disposed at the rear end of the bottom portion 312 and be coupled to an inner surface of the dispenser case 21.

An opening 312b may be defined in a center of the bottom portion 312 to dispose the ice chute 24, and the manipulation portion 313 extending downward along the circumference of the opening 312b may be provided. The manipulation portion 313 may be provided to surround the ice chute 24 from the front and may define the upper portion of the dispensing portion 27.

The manipulation portion 313 may extend downward to a length that is sufficient to provide the touch portion 316 and the display 315. In addition, an outer surface of the manipulation portion 313 may be provided in a curved shape and may be provided in a rounded shape so that a center thereof protrudes gradually backward toward both sides. The manipulation portion 313 may extend downward, and a lower end of the manipulation portion 313 may extend to the lighting assembly 40.

The manipulation portion 313 may include the touch portion 316 and the display 315. The touch portion 316 may be configured to induce the user's touch manipulation by printing or surface processing on the surface of the manipulation portion 313. In addition, the touch portion 316 may be provided at a position corresponding to a sensing member 35 of the touch device 32. Thus, when the user manipulates the touch portion 316, the sensing member 35 may recognize the user's touch manipulation.

The display 315 may be disposed above the touch portion 316 and may be provided at a position corresponding to the position of the light emitting member 331 provided in the touch device 32. The display 315 is configured to allow light to pass through, and therefore, when the light emitting member 331 is turned on, light irradiated from the light emitting member 331 may be transmitted and shined to display the user's touch manipulation or set state.

The display 315 may be configured to allow the light to pass therethrough. For this, the touch case 31 may be made of a plastic material that is capable of transmitting light. In addition, a blocking layer 314 that blocks light transmission may be provided on a remaining area except for the display 315. The blocking layer 314 may be provided in various forms such as coating, painting or film attachment, printing, and the like.

For example, the blocking layer 314 may be disposed on an entire surface of the touch case 31 through which light is transmitted, and the display 315 may locally remove the blocking layer 314 through laser processing or the like so that the light is transmittable. The display 315 may be provided in a very fine hole shape through the above processing. In addition, the display 315 may be provided in plurality per one touch portion 316. In addition, the display 315 may be provided in a plurality of spot shapes, may be provided in any shape, or may be provided in a specific shape as necessary.

In detail, after injection-molding the touch case 31 using the plastic material that is capable of transmitting light, a surface of the touch case 31 may be coated, painted, film-attached, printed, or sprayed to provide the blocking layer 314 that blocks the light transmission.

Here, the blocking layer 314 may be disposed on the entire surface of the touch case 31 through which light is transmitted, and the area of the touch case 31, on which the blocking layer 314 is disposed may be irradiated from the light emitting member 331.

After providing the blocking layer 314, the display 315 may be formed by peeling off a surface of the blocking layer 314 through laser processing, etc. so that the peeling-off portion is transparent. Also, when the light emitting member 331 is turned on, the light emitted from the light emitting member 331 may be guided to the rear surface of the display 315 to illuminate the display 315. In addition, the user may check the operation state and set state of the dispenser 20 through the display 315.

The display 315 may include at least one or more micro-holes 315a by the laser processing.

In addition, when the display 315 is provided in the shape having a plurality of micro-holes, a distance between the adjacent micro-holes may be larger than a diameter d2 of each of the micro-holes. When the micro-holes 315a are provided in plurality, the micro-holes 315a may overlap between the adjacent micro-holes 315a by diffusion of light passing through the micro-holes 315a. Here, an interval between the adjacent micro-holes is disposed to be larger than the diameter of each of the micro-holes so that the light passing through each of the micro-holes 315a is recognized more clearly. For example, a diameter d2 of each of the micro-holes may be about 0.3 mm to about 0.7 mm, and an interval between adjacent micro-holes may be about 1 mm to about 1.5 mm.

The blocking layer 314 may be provided to have the same or similar texture to the outer plate 11. For example, the blocking layer 314 may be provided in the same or similar color as the outer plate 11 so as to have a sense of unity with the outer plate 11 made of a metal material, and thus, the touch case 31 may provide an outer appearance that is more integrated with the door 10.

The display 315 may be provided in a fine size, and a degree of removal of the blocking layer 314 may be adjusted according to an output of the laser. Therefore, when the light emitting member 331 is turned off, the display 315 may not be well exposed to the outside, and the display 315 may be clearly identified from the outside only when the light emitting member 331 is turned on.

Of course, the display 315 may be integrated with the touch portion 316. That is, when the display 315 is provided on the area of the touch portion 316 to have the same shape as the touch portion 316, and the light emitting member 331 is turned on, the shape of the touch portion 316 may be shined brightly.

In addition, a case coupling protrusion 313a coupled to the lighting assembly 40 may be provided on the rear surface of the manipulation portion 313. The case coupling protrusion 313a may be provided at each of positions facing each other on both left and right surfaces. In addition, the case coupling protrusion 313a may be disposed at a position at which a curved front surface and both straight side surfaces of the manipulation portion 313 are in contact with each other. The case coupling protrusions 313a may protrude in opposite directions and may be provided to be hooked and restricted in a body restriction groove 412b to be described below.

In addition, a mounting portion hook 313b for the mounting the touch device 32 may be disposed on the rear surface of the manipulation portion 313. The mounting portion hook 313b may be coupled to a restriction groove 321 defined in an upper end of the touch device 32 to restrict the touch device 32. In addition, a screw coupling portion (not shown) to which a screw 36 for mounting the touch device 32 is coupled may be further provided on the rear surface of the manipulation portion 313. The screw 36 may pass through both left and right sides of the lower end of the touch device 32 so as to be coupled to the screw coupling portion. Thus, the touch device 32 may be fixed in a state of being in close contact with the rear surface of the manipulation portion 313 having a curved shape.

The touch device 32 may be configured to recognize the manipulation of the touch portion 316 and transmit light through the display 315 so as to display the manipulation state. The touch device 32 may include a touch substrate 33, on which a touch sensor 332 and the light emitting member 331 are mounted, and a guide member 34 mounted on a front surface of the touch substrate 33.

In detail, the touch substrate 33 may define a rear surface of the touch device 32, and the sensing member 35 may be mounted thereon. The sensing member 35 may be disposed at a position corresponding to the touch portion 316. In this embodiment, three sensing members 35 are provided as an example, but the number of sensing members 35 may be determined according to the configuration of the touch portion 316.

The sensing member 35 may include a touch sensor 332, an elastic member 351, and a transfer member 352. The touch sensor 332 may be provided as a capacitive touch sensor 332 and may be configured to detect a change in static electricity when the user touches the touch portion 316. In addition, the touch sensor 332 may be provided on the touch substrate 33 and may be electrically connected to the transfer member 352. For example, the touch sensor 332 may be provided on a rear surface of the diffuser touch substrate 33. Thus, even if the user manipulates the touch portion 316 disposed on the manipulation portion 313 provided in the curved shape, the elastic member 351 and the transfer member 352 may effectively detect the touch manipulation.

In detail, an elastic member 351 may be disposed in front of the touch sensor 332. The elastic member 351 may be made of an elastically deformable material such as sponge and may be provided in a block shape. In addition, the elastic member 351 may be provided to protrude further than a distance from the touch substrate 33 up to the manipulation portion 313. Thus, when the touch device 32 is mounted, the elastic member 351 may be completely in close contact with the rear surface of the manipulation portion 313 provided in a curved shape.

In detail, a plurality of elastic members 351 may be provided on the front surface of the touch device 32. The elastic member 351 may be mounted on the touch substrate 33, pass through the opening 32a defined in the front surface of the touch device 32, and be in contact with the rear surface of the manipulation portion 313.

For example, the elastic member 351 may be fixed to the front surface of the touch substrate 33 by soldering or the like on the touch substrate 33 and may extend so as to be in contact with the rear surface of the manipulation portion 313. That is, an edge portion that is in contact with the touch substrate 33 and the elastic member 351 may be fixed through a soldering operation.

The elastic member 351 may include a first elastic member 351a disposed at a center of the manipulation portion 313 and a pair of second elastic members 351b respectively disposed on both left and right sides of the first elastic member 351a, respectively.

In addition, a first touch sensor and a second touch sensor may be installed on the touch substrate 33 at positions corresponding to the first and second elastic members 351a and 351b, respectively.

The first elastic member 351a may be provided on a rear surface of the portion that most protrudes forward on the manipulation portion 313. That is, the touch device 32 may have a shape corresponding to that of the rear surface of the manipulation portion 313 and may be mounted on the most forward portion of the touch device 32.

In addition, a front surface of the first elastic member 351a may correspond to a shape of a central portion of the rear surface of the manipulation portion 313 so as to be in close contact with the rear surface of the manipulation portion 313. Alternatively, the front surface of the first elastic member 351a may have the same height on both left and right sides protruding forward from the opening 32a.

The second elastic member 351b may be provided to be spaced a predetermined distance from each other at left and right sides with respect to the first elastic member 351a. A position at which the second elastic member 351b is provided may be a position spaced apart from the center of the front surface of the touch device 32 to both sides.

The front surface of the second elastic member 351b may further include an inclined portion 351c to be in close contact with the rear surface of the manipulation portion 313.

In detail, the manipulation portion 313 is provided in a shape that is rounded backward toward both sides from the center. If the front surface of the second elastic member 351b has the same length as the left and right side of the first elastic member 351a, the entire front surface of the second elastic member 351b may not be in close contact with the rear surface of the manipulation portion 313 by the rounded portion of the manipulation portion 313. The entire surface of the manipulation portion 313 cannot be in close contact with the rear surface. In this case, there is a limitation in that touch sensitivity varies according to the position touched by the user.

Accordingly, the second elastic member 351b may have the inclined portion 351c that is inclined to further protrude forward in a direction away from the first elastic member 351a. That is, a pressure pushed by the rear surface of the manipulation portion 313 may more increase in a direction away from the first elastic member 351a, i.e., in an outward direction. Accordingly, it is possible to secure a uniform contact surface on the rear surface of the manipulation portion 313, and even if the user touches the manipulation portion 313 corresponding to the second elastic member 351b in a direction slightly off-center, the user's touch may be detected, and reliability may be improved.

Also, although not limited thereto, a length of the first elastic member 351a, which protrudes forward from the opening 32a of the touch device 32, may be about 1 mm to 4 mm, preferably about 1 mm to 3 mm. have. In addition, a length of the overlapping portion of the first elastic member 351a and the second elastic member, which protrudes forward from the opening 32a, is at least 2 mm or more.

In addition, a transfer member 352 may be provided on an outer surface of the elastic member 351. The transfer member 352 may be made of a material capable of transferring static electricity on the surface of the manipulation portion 313 to the touch sensor 332. In addition, the transfer member 352 may be provided to surround the outer surface of the elastic member 351 and may be made of a material that is capable of being deformed together when the elastic member 351 is deformed. Accordingly, the transfer member 352 may be made of a thin metal thin film or a conductive film material and may be provided to have a flexible structure.

The transfer member 352 may be disposed along the elastic member 351, and an end of the transfer member 352 may be connected to the touch substrate 33. Therefore, the rear surface of the manipulation portion 313 and the touch sensor 332 of the touch substrate 33 may be electrically connected to each other. For example, the transfer member 352 may be disposed to be connected along the front surface and both left and right surfaces of the elastic member 351, and a rear surface of the elastic member 351 may be mounted on the touch substrate 33 by welding or the like.

The touch substrate 33 may further include a light emitting member 331. The light emitting member 331 may be disposed above each of the sensing members 35. In addition, the light emitting member 331 may be configured so that the corresponding light emitting member 331 is turned on when the user's touch is sensed by the sensing member 35. The light emitting member 331 may be provided as, for example, an LED.

The guide member 34 may be provided on the front surface of the touch substrate 33. The guide member 34 may define a front surface of the touch device 32 and may be configured to be filled into a space between the touch substrate 33 and the rear surface of the manipulation portion 313 in a state in which the touch device 32 is mounted.

In addition, the guide member 34 may guide the light irradiated from the light emitting member 331 to the display 315. In addition, the guide member 34 may guide the sensing member 35 to be maintained in a mounted state at an accurate position.

In detail, a front surface of the guide member 34 may be provided in a curved shape corresponding to the rear surface of the manipulation portion 313, and when the touch device 32 is mounted, the front surface of the guide member 34 may be completely in close contact with the rear surface of the manipulation portion 313. In addition, a circumference of the guide member 34 may extend backward and may be fixedly mounted on the front surface of the touch substrate 33.

In addition, a hook coupling portion 343 to which the mounting portion hook 313b is coupled may be disposed on a top surface of the guide member 34. In addition, the screw 36 may pass through the lower end of the touch substrate 33, and the screw 36 may be coupled to the screw coupling portion so that the touch device 32 is fixed and mounted in a state of being in close contact with the rear surface of the manipulation part 313. It may be fixed and installed in a fixed state.

A guide hole 341 may be defined in the guide member 34. The guide hole 341 may be opened at a position corresponding to the touch portion 316 and the sensing member 35 and may define a space in which the sensing member 35 is accommodated. In addition, the guide hole 341 may have a cross-sectional shape corresponding to the sensing member 35, and even if the elastic member 351 is elastically deformed, the sensing member 35 may be prevented from being deviated from the arrangement position or abnormally deformed.

That is, the sensing member 35 may protrude further forward than the opened front surface of the guide hole 341 in a state in which the sensing member 35 is mounted on the touch substrate 33, and when the touch device 32 is mounted, the front surface of the sensing member 35 may be deformed to be in contact with the rear surface of the manipulation portion 313. Here, the sensing member 35 may be pressed in the guide hole 341 to maintain a position at which the manipulation of the touch portion 316 is accurately detected.

In addition, a light hole 342 may be defined above the guide hole 341. The light hole 342 is configured to guide the light emitted from the light emitting member 331 to the display without leaking to the outside, and the light hole 342 may extend from the front surface of the touch substrate 33 to the rear surface of the manipulation portion 313. In addition, the light emitting member 331 may be disposed inside the guide hole 341. The light hole 342 may provide a passage through which light is irradiated between the display 315 and the light emitting member 331.

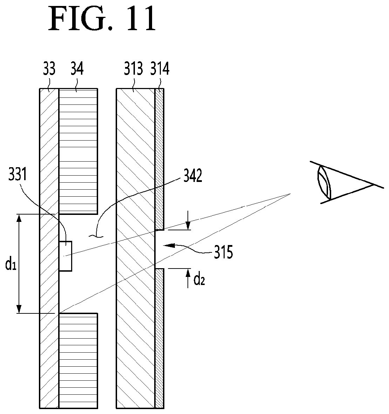

Fig. 11 is a schematic view illustrating a viewing angle of the user when the user sees the touch assembly at the front.

Referring to Fig. 11, a vertical length d1 of the light hole 342 may be greater than a vertical length d2 of the display 315.

In addition, a center line of the display 315 may be defined at a position corresponding to an upper end of the light hole 342. That is, the display 315 may be disposed at a position closer to the upper end of the light hole 342 than the lower end. In other words, a length between the upper end of the display 315 and the upper end of the light hole 342 may be less than a length between the lower end of the display 315 and the lower end of the light hole 342.

When the user stands in front of the refrigerator and looks at the display 315, a height of the user's eyes may be disposed higher than the display 315. If the user looks at the display 315, and a viewing angle is lower than the light emitting member 331, the light passing through the display 315 may not be properly recognized.

When the display 315 is disposed close to a position corresponding to the upper end of the light hole 342, the viewing angle of the user looking at the upper side is disposed between the light emitting member 331 and the lower end of the light hole 342. In this case, the user's viewing angle is not disposed below the light hole 342, and thus, the entire light passing through the display 315 may be recognized. Thus, the user may easily recognize the light passing through the display 315 on the touch assembly 30 provided as the curved surface.

With this structure, when the user touches the touch portion 316, the sensing member 35 may detect the user's touch, and the light emitting member 331 corresponding to the sensing member 35 may be turned on to irradiate light to the display 315, thereby illuminating the display 315. In addition, the user may check the operation state and the set state of the dispenser 20 through the display 315.

Hereinafter, the light assembly 40 and constituents adjacent to the light assembly 40 will be described in more detail with reference to the drawings.

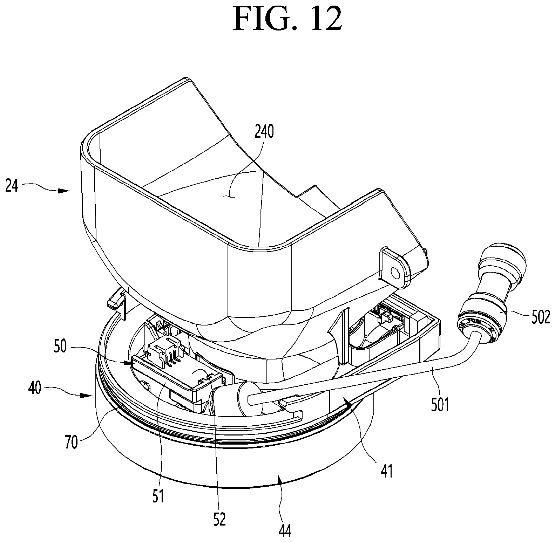

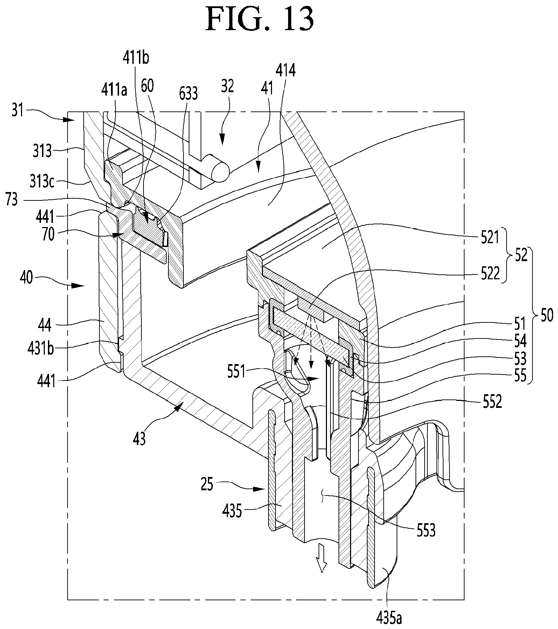

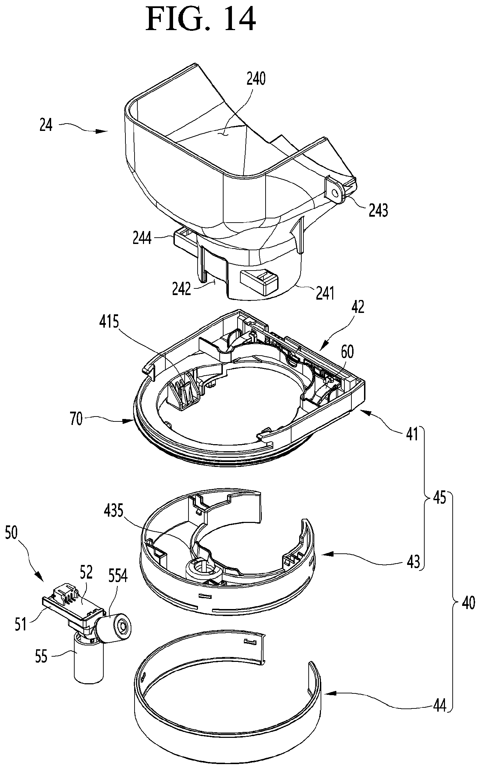

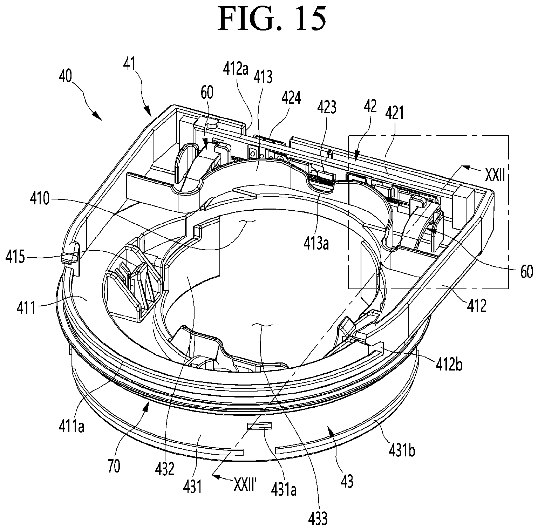

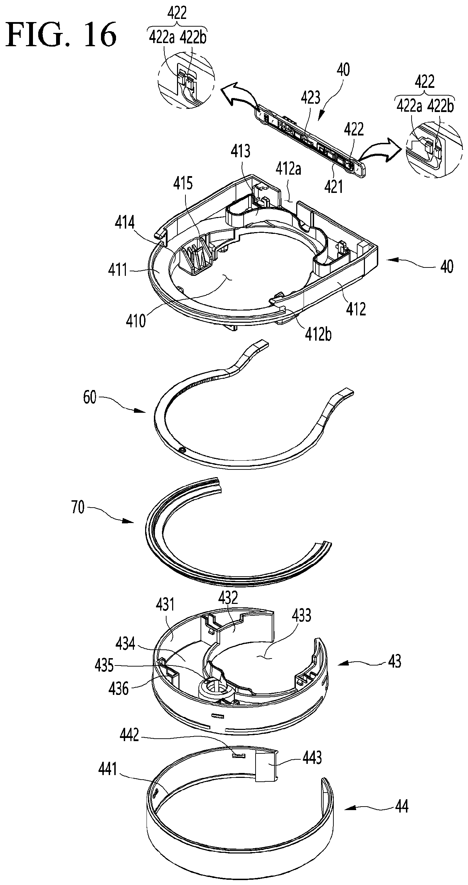

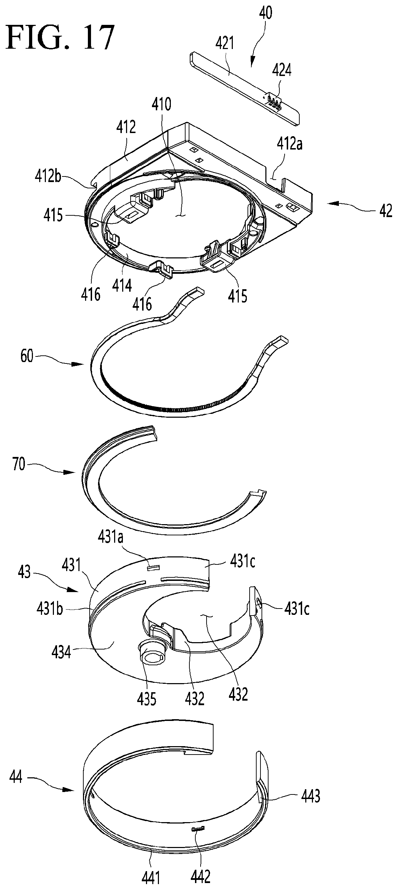

Fig. 12 is a perspective view illustrating a state in which a lighting assembly, which is one component of the dispenser, the ice chute, and a nozzle assembly are coupled to each other. Also, Fig. 13 is a cross-sectional view taken along line XIII-XIII' of Fig. 3. Also, Fig. 14 is an exploded perspective view of the lighting assembly, the ice chute, and the nozzle assembly. Also, Fig. 15 is a perspective view of the lighting assembly. Also, Fig. 16 is a front exploded perspective view illustrating a state in which the lighting assembly is disassembled. Also, Fig. 17 is a bottom exploded perspective view illustrating the state in which the lighting assembly is disassembled.

As illustrated in the drawings, the lighting assembly 40 may be provided in a ring shape, and a rear end of the lighting assembly 40 may be fixedly mounted to the dispenser case 21. In addition, the ice chute 24 and the nozzle 55 may be mounted on the lighting assembly 40.

The ice chute 24 may be provided in a vertically opened shape, and a space 240, through which ice discharged through the connection passage 23 passes, may be defined therein. In addition, the opened upper end of the ice chute 24 may be opened toward the connection passage 23 and may be provided to be greater than that of the end of the connection passage 23. In addition, an upper portion of the ice chute 24 disposed above the lighting assembly 40 may have a diameter greater than that of a lower portion of the ice chute 24.

An upper fixing portion 243 protruding laterally and fixed to the inner surface of the dispenser case 21 may be disposed on each of both left and right surfaces of the upper portion of the ice chute 24. In addition, a lower fixing portion 244 protruding laterally and fixed to both sides of the lighting assembly 40 may be disposed on each of left and right surfaces of the lower portion of the ice chute 24. The ice chute 24 may be maintained at a mounting position in the dispenser 20 by the upper fixing portion 243 and the lower fixing portion 244.

The lower portion of the ice chute 24 may extend through the lighting assembly 40 and may extend further downward than the lower end of the lighting assembly 40. In addition, a chute outlet 241 at the lower end of the ice chute 24 may face downward, and the lower end of the ice chute 24 may be exposed below the lighting assembly 40.

The lighting assembly 40 may be made of a material capable of being transparent or transmissive and may be made of a material capable of diffusing light. Therefore, when a chute light emitting member 331, which will be described below, is turned on, the ice chute 24 itself may be illuminated. That is, at least the lower end of the ice chute 24 exposed downwards of the lighting assembly 40 is illuminated, and thus, the user may visualize and indicate the use state of the dispenser 20.

In addition, a chute groove 242 may be defined in a lower end of a front surface of the ice chute 24. The chute groove 242 may be provided in the form of a groove recessed upward from the lower end and may be provided to accommodate a portion of the water outlet 25 of the lighting mounting portion on which the nozzle 55 is mounted.

That is, at least a portion of the lower end of the ice chute 24 and the lower end of the water outlet 25 may overlap each other, and thus, the cup or container of the dispenser 20 may accommodate the water and ice in a state of being at the same position or not largely moved.

The nozzle assembly 50 may be mounted in front of the ice chute 24.

The nozzle assembly 50 is configured to allow purified water to be dispensed and may be fixedly mounted inside the lighting assembly 40 and be inserted and mounted inside the nozzle insertion portion 435 formed in the lighting assembly 40.

The nozzle assembly 50 includes a nozzle 55 from which purified water is discharged, a sterilization device 52 provided in the nozzle 55 to sterilize water passing through the nozzle 55, a tube 501 connected to the nozzle 55, and a fitting 502.

An inlet 551 through which purified water is introduced may be provided at an upper portion of the nozzle 55. The inlet 551 may communicate with a tube connection portion 554 disposed at an upper end of the nozzle 55. The tube 501 through which purified water flows may be connected to the tube connection portion 554. Accordingly, water introduced through the tube connection portion 554 may be supplied to the inlet 551.

In addition, the inlet 551 may have an open top surface, and the sterilization device 52 may be disposed on the opened top surface of the inlet 551. The sterilization device 52 may be configured to sterilize the inside of the nozzle 55 by irradiating ultraviolet rays to the inside of the nozzle 55.

The sterilization device 52 may include a sterilization substrate 521 on which a sterilization LED 522 irradiating ultraviolet rays is mounted. In addition, the sterilization device 52 may be fixedly mounted to a sterilization case 51 mounted on an upper end of the nozzle 55. The sterilization case 51 may be coupled to an upper end of the inlet 551, and the opened top surface of the inlet 551 may be shielded by the sterilization case 51 and the sterilization substrate 521. In a state in which the sterilization substrate 521 is mounted, the sterilization LED 522 may be disposed to face the inside of the inlet 551. In addition, the sterilization LED 522 may be disposed on the same extension line as the water outlet passage 553 inside the nozzle 55 to sterilize the inlet 551 as well as the water outlet passage 553 through which water flows.

The sterilization device 52 may include a light transmission cover 53. The light transmission cover 53 may be mounted to shield the opened top surface of the inlet 551 and may shield the sterilization LED 522 in the mounted state. Thus, the water of the inlet 551 may be blocked from flowing into the sterilization LED 522 or the sterilization substrate 521. Of course, the light transmission cover 53 may be provided so that ultraviolet rays irradiated from the sterilization LED 522 are transmitted. For example, the light transmission cover 53 may be made of glass or a transparent acrylic material.

A sealer 54 may be further provided around the light transmission cover 53, and water may be prevented from leaking between the light transmission cover 53 and the nozzle 55 or the sterilization case 51 by the sealer 54.

The nozzle 55 may extend downward and be inserted into the nozzle insertion portion 435. The nozzle 55 may extend to a lower end of the nozzle insertion portion 435. In addition, an outlet water passage 553 may be provided to pass from the lower end of the inlet 551 to the lower end of the nozzle 55. Thus, water of the inlet 551 may be discharged to the lower end of the nozzle 55 through the water outlet 553.

A guide rib 552 may be disposed inside the nozzle 55. The guide rib 552 may protrude from an inner surface of the inlet 551 toward a center and may pass through the inlet 551 to extend up to the inside of the outlet passage 553. The guide rib 552 may allow the water introduced from the side through the tube connection portion 554 to pass through the outlet passage 553 more effectively without being attached to an inner wall surface of the inlet 551. A plurality of guide ribs 552 may be provided in a radial direction.

The lighting assembly 40 may include an upper body 41 and a lower body 43 to which the light guide 60 and the diffusion member 70 are mounted. The upper body 41 and the lower body 43 may be coupled to each other, and the upper body 41 and the lower body 43 may be referred to as a dispensing portion body 45. In addition, the lower body 43 may be provided with a lower cover 44 forming an outer appearance thereof.

In addition, the ice chute 24 may be fixedly mounted to the upper body 41. A body coupling portion 415 coupled to the lower fixing portion 244 of the ice chute 24 may be provided inside the upper body 41. The body coupling portion 415 may be provided in a shape such as a hook and may be hooked and restricted by the lower fixing portion 244.

In addition, the nozzle assembly 50 may be fixedly mounted to the lower body 43. The nozzle inserting part 435 protruding downward may be provided on a bottom surface of the lower body 43, and the nozzle insertion portion 435 may be penetrated in the vertical direction so that the nozzle 55 is inserted. The nozzle insertion portion 435 may have a cylindrical shape and may extend up to a height corresponding to the lower end of the ice chute 24.

In addition, a nozzle decoration 235a may be provided on an outer surface of the nozzle insertion portion 435. The nozzle decoration 235a may be made of a metal material or a material having a metal texture and may be provided in a cylindrical shape so as to be inserted into the outer surface of the nozzle insertion portion 435.

A portion which protrudes downward, from which water is dispensed, and which includes the nozzle insertion portion 435, the nozzle decoration 235a, and the nozzle 55 may be referred to as a water outlet 25. Of course, the water outlet 25 may be configured only with at least one of the nozzle insertion portions 435 and the nozzle 55.

The light guide 60, a lighting module 42 that irradiates light to the light guide 60, and a diffusion member 70 that diffuses the light of the light guide 60 so as to be seen to the outside may be mounted on the upper body 41.

In a state in which the touch assembly 30 and the lighting assembly 40 are mounted, an outer end of the light guide 60 may be exposed between the touch assembly 30 and the lighting assembly 40, and when an LED 422 of the lighting module is turned on, the diffusion member 70 may be shined in a ring shape from the outer surface of the water outlet 25. The light guide 60 and the diffusion member 70 may be coupled to each other, and the light guide 60 and the diffusion member 70 may be referred to as a light ring.

Hereinafter, a structure of the lighting assembly will be described in more detail with reference to the accompanying drawings.

Fig. 11 is a perspective view of the lighting assembly, Fig. 12 is a perspective view illustrating a state in which the lighting assembly, which is one component of the dispenser, the ice chute, and the nozzle assembly are coupled to each other, and Fig. 13 is a cross-sectional view taken along line XIII-XIII' of Fig. 3.

As illustrated in the drawings, the lighting assembly 40 may include the lighting module 42, the upper body 41, the lower body 43, and the light guide 60. In addition, the lighting assembly 40 further includes the diffusion member 70.

The lighting module 42 may include an LED 422 irradiating light to the light guide 60 and a light substrate 421 on which the LED 422 is mounted. The light substrate 421 may have a horizontal length greater than that of the light guide 60. In addition, the LED 422 may be provided on each of both sides of the light substrate 421 to radiate light toward both rear ends of the light guide 60.

The LEDs 422 may be disposed to be symmetrical on both left and right sides with the light substrate 421 as the center and may be spaced apart from each other at the left and right sides based on the position of the nozzle 55.

In addition, a plurality of the LEDs 422 may be provided at both left and right sides, respectively. In this case, all of the plurality of LEDs 422 may be disposed between both ends of the rear end of the light guide 60. That is, a distance between the outer ends of the plurality of LEDs 422 may be provided to be less than a width of the rear end of the light guide 60. Accordingly, the light irradiated from the plurality of LEDs 422 may be directed toward the rear end of the light guide 60 as a whole.

In addition, the plurality of LEDs 422 may have different colors. For example, the plurality of LEDs 422 may include a blue LED 422a and a white LED 422b. Thus, information to be displayed through the light emission of the ring light 730 may be displayed through the color of the LED 422. That is, the ring light 730 may emit light in different colors depending on the operation state or set state of the dispenser 20 or the refrigerator 1.

Various elements or light emitting bodies capable of irradiating light may be used as the LED 422 and thus may be referred to as a light source or a light emitting member. Also, the LED 422 is not provided as a plurality of light sources shining in each color but may be provided as a single component. Also, the light source provided as the single component may be configured to operate so as to shined in a plurality of colors.

The light substrate 421 may further include a suit LED 423. The chute LED 423 may irradiate light toward the ice chute 24 and may be disposed between the light substrate 421 and the lighting assembly 40. The chute LED 423 may be disposed behind the nozzle 55 and may be disposed on the same extension line in the front and rear direction as the nozzle 55.

When the suit LED 423 is turned on, light may be irradiated toward a middle portion of the outer surface of the ice suit 24. Thus, when the suit LED 423 is turned on, the lower end of the ice suit 24 exposed downward of the lighting assembly 40 may be brightly illuminated, and the operation of the dispenser 20 may be visualized to the user.

The upper body 41 may be entirely made of plastic injection molding and may have a structure in which the ice chute 24, the lighting module 42, and the light guide 60 are mounted.

The lighting module 42 may further include a lower cover 44. The lower cover 44 may be configured to define an outer appearance exposed to the outside of the lighting module 42 and may be mounted on an outer surface of the lower body 43.

In detail, the upper body 41 may include an upper base 411 defining a bottom surface and an upper opening 410 passing through a center of the upper base 411 so that the ice chute passes therethrough. In addition, a body coupling portion 415 may be provided on each of both left and right sides of the upper opening 410 and may have a coupling structure with the lower fixing portion 244 of the ice chute 24.

In addition, an upper edge 412 may be disposed along an outer circumference of the upper base 411. The upper edge 412 may extend upward. The upper edge 412 may define a space in which the lighting module 42 is mounted, while providing the coupling structure with the touch assembly 30.

The upper edge 412 may be disposed on each of both sides and a rear surface of the entire circumference of the upper base 411. The upper edge 412 may have a height equal to or greater than that of the upper end of the lighting module 42 so that the lighting module 42 provided therein does not interfere with other components. In addition, the upper edge 412 may have a height that is capable of being inserted into the lighting assembly mounting portion 217.

A restriction grooves 412b may be defined in a front end of each of both sides of the upper edge 412. The body restriction groove 412b may be opened forward so as to be inserted while moving from the front to the rear with the case coupling protrusion 313a. Since the case coupling protrusion 313a is coupled to the body restriction groove 412b, the touch assembly 30 and the lighting assembly 40 may be firmly coupled to each other.

A stepped portion 411a protruding upward along the front end of the upper base 411 in which the upper edge 412 is not formed may be further provided, and the touch case 411a, that is, a lower end of a curved front surface of the manipulation portion 313 may be supported by the stepped portion 411a.

In addition, a connector groove 412a may be defined in one rear side of the upper edge 412. The connector groove 412a may be a portion at which a cable connector 424 provided in the light substrate 421 is exposed, and when the lighting module 42 is assembled and mounted, the cable harness easily extends to the cable connector 424. In addition, the cable harness connected to the cable connector 424 may be disposed inside the lighting assembly mounting portion 217.

The upper body 41 may be provided with a barrier 413 that provides a space for the mounting of the lighting module 42. The barrier 413 may partition the inner space of the upper body 41 back and forth. That is, the barrier 413 forms a space at the rear of the ice chute 24 in which the lighting module 42 is mounted.

In detail, the barrier 413 may extend upward from the top surface of the upper base 411 and may be provided along a circumference of the second half of the upper opening 410. In addition, both ends of the barrier 413 may be connected to both sides of the upper edge 412. Thus, a space may be defined between the upper opening 410 and the rear surface of the upper edge 412 by the barrier 413.

The barrier groove 413a may be defined in the barrier 413. The barrier groove 413a is recessed at a position facing the chute LED 423, and thus, most of the light irradiated from the chute LED 423 may not interfere with the barrier 413 but directed to the ice chute 24. The barrier groove 413a may be formed at an intermediate position of the barrier 413.

Also, a substrate restriction portion 418, side surface fixing portion 12c and 41d, and a substrate support 412e, which support and fix the light substrate 421, and guide restriction portions 416 and 417 that fixes the light guide 60 may be provided inside a space defined by the barrier 413. Structures of the substrate restriction portion 418 and the guide restriction portions 416 and 417 will be described below in detail.

The upper body 41 may include a support 414. The support 414 may protrude downward from the lower surface of the upper base 411 and may be configured to support the light guide 60 from the rear. The light guide 60 may be disposed in a fixed position by the support 414, and the mounted state may be maintained.

The support 414 may be disposed along a portion of a circumference of the upper opening 410 and may extend downward. The support 414 may extend further downward than a lower end of the light guide 60. In addition, as illustrated in FIG. 9, the support 414 may further extend downward by passing through the light guide 60 and the diffusion member 70. That is, a lower end of the support 414 may be disposed further below the lower ends of the light guide 60 and the diffusion member 70 and may support both the light guide 60 and the diffusion member 70.

The support 414 may have a curvature corresponding to that of the round portion 63 of the light guide 60 and may be provided to support the entire rear surface of the round portion 63 from the rear.

As illustrated in Fig. 9, a base restriction groove 411b guiding a mounting position of the light guide 60 may be disposed on a lower surface of the upper base 411 in front of the support 414. The base restriction groove 411b may be recessed to accommodate the guide protrusion 633 protruding from the top surface of the light guide 60 and may allow the light guide 60 to be maintained in a mounting position.

An upper coupling portion 416 may be disposed at a lower end of the support 414. A plurality of upper coupling portions 416 may be provided along the support 414 and may protrude downward. In addition, the upper coupling portion 416 may be provided at a position corresponding to the lower coupling portion 436 disposed on the lower body 43 and may be coupled to the lower coupling portion 436. Accordingly, the upper body 41 and the lower body 43 may be firmly coupled by the coupling of the upper coupling portion 416 and the lower coupling portion 436.

The light guide 60 may be mounted on a lower surface of the upper body 41, and a rear end of the light guide 60 may be disposed at a position corresponding to the LED 422. Thus, when the LED 422 is turned on, the irradiated light may move along the light guide 60.

In addition, the diffusion member 70 may be provided on a lower surface of the upper body 41. The diffusion member 70 may be coupled to the light guide 60, and the light irradiated from the light guide 60 may be diffused and visible from the outside. For this, an outer end of the light guide 60 may be exposed between the upper body 41 and the lower body 43, and light may be irradiated to the outside.

The lower body 43 may support the light guide 60 from below and may be coupled to the upper body 41 under the upper body 41. The lower body 43 may be provided to be rounded at a curvature corresponding to that of each of the upper body 41 and the manipulation portion 313. In addition, the lower body 43 may be provided in a cylindrical or ring shape of which a portion of the rear end is cut. The lower body 43 may be injection-molded from a plastic material and may have a structure that is capable of mounting the nozzle assembly 50, supporting the diffusion member 70, and being coupled to the upper body 41.

In detail, the lower body 43 may include a lower base 434 defining a bottom surface. In addition, a lower opening 433 may be defined in the lower base 434 to allow the ice chute 24 to pass therethrough. In addition, the nozzle insertion portion 435 in which the nozzle 55 is mounted may be disposed at one side of the lower base 434 in front of the lower opening 433. The nozzle insertion portion 435 may extend below the lower base 434 and may be provided to be penetrated vertically so that the nozzle 55 is inserted.

A lower edge 431 extending upward along the lower body 43 may be disposed at an outer end of the lower body 43. The lower edge 431 may extend upward to support the diffusion member 70 from below.

In addition, an inner support 432 may be provided along a circumference of the lower opening 433 defined in the lower base 434. The inner support 432 may extend upward along the circumference of the lower opening 433, and the inner support 432 may surround and support the outer surface of the ice chute 24.

The lower coupling portion 436 coupled to the upper coupling portion 416 of the upper body 41 may be disposed on the inner surface of the lower edge 431. In addition, an edge groove 431a and an edge protrusion 431b for the coupling of the lower cover 44 may be disposed on an outer surface of the lower edge 431. The edge groove 431a may be defined along an upper portion of an outer surface of the lower edge 431, and a plurality of edge grooves 431a may be disposed at regular intervals. In addition, the edge protrusion 431b may protrude outward along the lower portion of the outer surface of the lower edge 431 and may protrude at a position spaced apart from the lower end of the lower edge 431. In addition, a rear end coupling portion 431c coupled to a rear end of the lower cover 44 may be further provided at the rear end of the lower edge 431.

The lighting module 42 may further include the lower cover 44. The lower cover 44 is configured to define an outer appearance exposed to the outside of the lighting module 42 and may be mounted on the outer surface of the lower body 43.

The lighting module 42 may have a size corresponding to the lower edge 431 of the lower cover 44 and may shield the lower edge 431 in a state of being mounted on the lower body 43. Thus, the outer appearance of the lower body 43, i.e., the outer appearance of the lighting module 42 exposed to the outside may be defined by the lower cover 44.

A cover protrusion 442 coupled to the edge groove 431a may be disposed on the rear surface of the lower cover 44, and a cover restriction protrusion 441, which is hooked and restricted with the edge protrusion 431b, may be disposed below the cover protrusion 442. A protrusion 441 may be formed. In addition, a cover coupling portion 443 coupled to the rear end coupling portion 431c may be further disposed at the rear end of the lower cover 44.

Accordingly, in the lower cover 44, the cover protrusion 442 may be coupled to the edge groove 431a, the cover restriction protrusion 441 may be hooked and restricted with the edge protrusion 431b, and the cover coupling portion 443 may be firmly coupled to the lower body 43 by being coupled to the rear end coupling portion 431c.

An outer surface of the lower cover 44 may have a film attached thereto, coated, or painted so that the outer appearance of the lower cover 44 is more conspicuous. For example, the outer surface of the lower cover 44 may be coated with a metal texture. In addition, an outer surface of the lower cover 44 may have the same color or texture as the outer plate 11.

Hereinafter, the structure of the light guide 60 and the diffusion member 70 will be described in more detail with reference to the drawings.

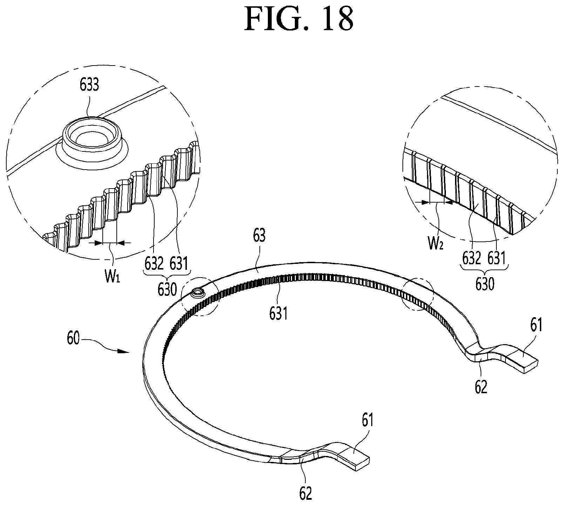

Fig. 18 is a perspective view of the light guide that is one component of the lighting assembly.

As illustrated in the drawing, the light guide 60 may be made of a material capable of transmitting light. The light guide 60 may be made of acrylic or plastic resin. In addition, the light guide 60 may be made of various materials capable of uniformly transmitting the irradiated light and may be configured to contain a light transmission material as an additive. Also, the light guide 60 may be provided in a ring shape as a whole and may be provided in a shape of which one side is cut.

The light guide 60 may include an extension portion extending forward from a position corresponding to each of both the LEDs 422 and a round portion 63 seated on the support of the upper body 60 and provided in a ring shape. In addition, the light guide 60 may include a connection portion 62 connecting an end of the round portion 63 and the extension portion 61. The round portion 63, the connection portion 62, and the extension portion 61 may be integrally provided by injection molding.

A cross-section of a rear end of the extension portion 61 may have a rectangular shape, and a width of the rear end of the extension portion 61 may be greater than a width of each of the pair of LEDs 422. Accordingly, the light irradiated from the LED 422 may be effectively supplied toward the rear end of the extension portion 61. In addition, the extension portion 61 may extend forward by a predetermined length, and the rear end of the extension portion 61 may extend to a position that is very close to the front surface of the LED 422.

A connection portion 62 may be disposed at the front end of the extension portion 61. The connection portion 62 may extend to an end of the round portion 63 and may be disposed to be inclined or rounded to face downward and laterally as it extends forward. In this case, the connection portion 62 may extend to pass through the upper body 41. That is, based on the upper base 411 of the upper body 41, the extension portion 61 may be disposed above the upper base 411, that is, in an inner space of the upper body 41, and the round portion 63 may be disposed below the upper base 411.

The round portion 63 may be provided in a ring shape and may be provided so that a portion of the second half is cut off. In addition, the cut rear end of the round portion 63 may be connected to the front end of the connection portion 62. In addition, the cross-section of the round portion 63 may be provided in a rectangular shape or a shape having four sides. That is, the top surface of the round portion 63 may be in contact with the lower surface of the upper base 411, and the lower surface of the round portion 63 and the front surface of the round portion 63, i.e., an outer surface of the round portion 63 may be in contact with the diffusion member 70, and the rear surface of the round portion 63 may be in contact with the support 414. Therefore, the round portion 63 may have a stable mounting structure between the upper body 41 and the diffusion member 70 and have a structure that is capable of effectively transmitting light through the close contact structure with the diffusion member 70.

The round portion 63 may be disposed further lower than the extension portion 61 and be in contact with the bottom surface of the upper base 411 in a state in which the light guide 60 is mounted on the upper body 41.

In addition, the round portion 63 may have a curvature corresponding to the rounded shape of the first half of the upper body 41. Accordingly, the round portion 63 may be disposed along a circumference of a front end of the upper body 41.

A guide protrusion 633 may be disposed at a center of a top surface of the round portion 63. The guide protrusion 633 may be inserted into the base restriction groove 411b recessed from the bottom surface of the upper base 411 when the light guide 60 is mounted. Thus, movement of the light guide 60 may be prevented by the coupling of the guide protrusion 633 and the base restriction groove 411b, and the light guide 60 may be induced to be mounted at an accurate position. The guide protrusion 633 may be a portion of a gate defined when a material is injected during injection molding of the light guide 60.

A pattern portion 630 may be disposed on the rear surface of the round portion 63, that is, on an inner circumferential surface of the round portion 63 facing the support 414. The pattern portion 630 may be a plurality of unevenness that are provided continuously so that the light is directed to the outside direction (a direction away from the center of the round portion 63) including the front of the light moving along the light guide 60.

In detail, in the pattern portion 630, a plurality of grooves 631 and protrusions 632 may be continuously and repeatedly disposed. Thus, the light irradiated through the round portion 63 may be reflected by the shape of the grooves 631 and the protrusions 632 of the pattern portion 630 to be reflected in the outward direction including the front. The protrusions 632 are provided to protrude relatively compared to the grooves 631 and may be provided in a planar shape, and all of the protrusions 631 on the pattern portion 630 may have the same height.

The plurality of grooves 631 constituting the pattern portion 630 are provide so that a width W1 gradually increases from the rear of the round portion 63, that is, from the portion connected to the connection portion 632 toward the front. In addition, the groove 631 may be provided to gradually increase in depth from the rear of the round portion 63, that is, from the portion connected to the connection portion 62 toward the front. In addition, the protrusions 632 between the adjacent grooves 631 may be provided to gradually increase in width W2 toward the front from the rear of the round portion 63, that is, from the portion connected to the connection portion 62.

Accordingly, light reflection may be reduced toward the rear of the round portion 63 closer to the LED 422, and the center of the round portion 63 furthest from the LED 422 has a relative light reflection to be more active, so that light is irradiated outward with uniform brightness throughout the round portion 63.

The shape (width and depth) of the grooves 631 and the protrusions 632 may be changed gradually depending on the positions of the respective grooves 631 and the protrusions 632, and a plurality of the grooves 631 and the protrusions 632 having the same shape, and each of the grooves 631 and the protrusions 632 may be provided in stages. In addition, the grooves 631 and the protrusions 632 in a predetermined area may have the same shape, and the shapes of the grooves 631 and the protrusions 632 may be different for each area.

Fig. 19 is a perspective view of the diffusion member that is one component of the lighting assembly.

As illustrated in the drawing, the diffusion member 70 diffuses the light irradiated forward through the light guide 60 in the process of passing through the diffusion member 70 so as to be shined evenly and brightly as a whole when viewed from the outside.

That is, the light irradiated to the front and the outside by the pattern portion 630 is inevitably partially shaded due to the shape of the grooves 631 and the protrusions 632, and in some cases, these shades are exposed to the outside. However, the light irradiated to the outside by the pattern portion 630 may be diffused as a whole in the process of passing through the diffusion member 70 so that the diffusion member 70 may be shined with a uniform brightness as a whole.

Thus, a portion of the diffusion member 70 exposed between the upper body 41 and the lower body 43 may emit light in a ring shape with uniform brightness, and the user may view the front and side surfaces of the dispenser 20. It is possible to see the ring light 730 of uniform brightness no matter where it is viewed.

For this, the diffusion member 70 may be made of a material capable of transmitting light and may be provided by adding a diffusion agent for light diffusion therein.

In addition, the diffusion member 70 may be provided in a ring shape having a curvature corresponding to the shape of the round portion 63 and may be provided so that a portion of the second half is cut like the round portion 63. The diffusion member 70 may support the light guide 60 and be constrained to the ends of the upper body 41 and the lower body 43, respectively, and may have a structure in which a portion is capable of being exposed to the outside.

In detail, the diffusion member 70 may include a diffusion member base 71 defining a bottom surface of the diffusion member 70, a front extension portion 72 protruding upward from the front end of the diffusion member base 71, and an exposed portion 73 protruding forward from the front extension portion 72 and exposed through between the upper body 41 and the lower body 43.

In detail, the diffusion member base 71 may be provided in a shape corresponding to the round portion 63, may have a predetermined width, and may be configured to be supported by the upper end of the lower edge 431. In addition, a seating surface 711 may be disposed on the top surface of the diffusion member base 71. The seating surface 711 has a lower surface of the round portion 63 seated therein and may be in surface contact with the entire lower surface of the round portion 63.

In addition, the front extension portion 72 may extend vertically upward from the front end of the diffusion member base 71. In this case, the front extension portion 72 may protrude to a height corresponding to the top surface of the round portion 63 in a state in which the round portion 63 is seated on the seating surface 711.

Accordingly, the front and lower surfaces of the round portion 63 are disposed to be in contact with the seating surface 711 and the front extension portion 72, respectively, so that a stable mounting state can be maintained. In particular, the front surface of the round portion 63 and the inner surface of the front extension portion 72 are in close contact with each other, and the light irradiated to the front and the outside through the round portion 63 is effectively applied to the front extension portion 72) and the exposed portion 73 may be provided to pass through.

The exposed portion 73 may protrude outward from the outer surface of the front extension portion 72. The exposed portion 73 may protrude along a circumference of the front extension portion 72 and may protrude forward even with a predetermined width. In addition, the exposed portion 73 may extend in a direction crossing the front extension portion 72 and may extend outward at a height corresponding to the round portion 63.

In addition, an inclined surface 721 may be disposed between the front extension portion 72 and the exposed part 73, and the inclined surface 721 may be in contact with the front end of the upper base 411 and the diffusion member 70 and thus may be fixed more firmly.

In addition, a stepped cover seating portion 731 may be disposed on the lower surface of the exposed portion 73 and the front surface of the front extension portion 72, and when the diffusion member 70 is mounted, an upper end of the lower cover 44 may be seated on the cover seat 731.

When the diffusion member 70 is seated on the lower body, the lower surface of the exposed portion 73 may be supported by the upper end of the lower cover 44. In addition, the top surface of the exposed portion 73 may be provided to be in contact with the lower end of the touch case 31. In addition, the exposed portion 73 may be disposed on the inner side of the outer surface of the manipulation portion 313 and the outer surface of the lower cover 44 and may be disposed at a relatively less protruding position.

Also, as illustrated in FIG. 9, a lower inclined surface 313c is provided at the lower end of the manipulation portion 313 toward the rear as it extends downward, and the upper end of the lower cover 44 has a rear as extending upward. An upper end inclined surface 441 that face a rear side as extending upward may be formed. The extending end of the exposed portion 73 may protrude to positions corresponding to the lower end of the lower inclined surface 313c and the upper end of the upper inclined surface 441.

Therefore, in a state in which the touch assembly 30 and the lighting assembly 40 are coupled, the coupling portion of the light guide 60 and the diffusion member 70 may not exposed, and only a portion of the diffusion member 70 is visible. That is, the exposed portion 73 may be seen as a ring shape between the touch assembly 30 and the lighting assembly 40 and may be disposed between the lower inclined surface 313c and the upper inclined surface 441 to be more visible.

As described above, when the upper body 41 and the lower body 43 are coupled, the light guide 60 and the diffusion member 70 may be disposed between the upper body 41 and the lower body 43, and the light guide 60 and the diffusion member 70 may emit light in a ring shape by the lighting module, so that the exposed portion 73 is finally visualized to the outside. In addition, a portion emitted to the outside by the exposed portion 73 may be referred to as a ring light 730.

Hereinafter, the structure for mounting the light guide 60 and the diffusion member 70 will be described in more detail with reference to the drawings.

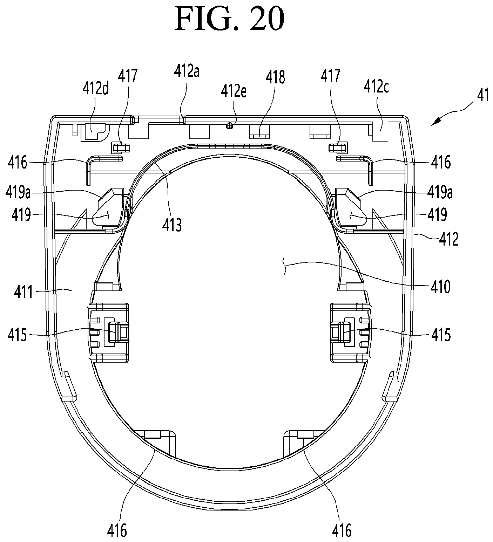

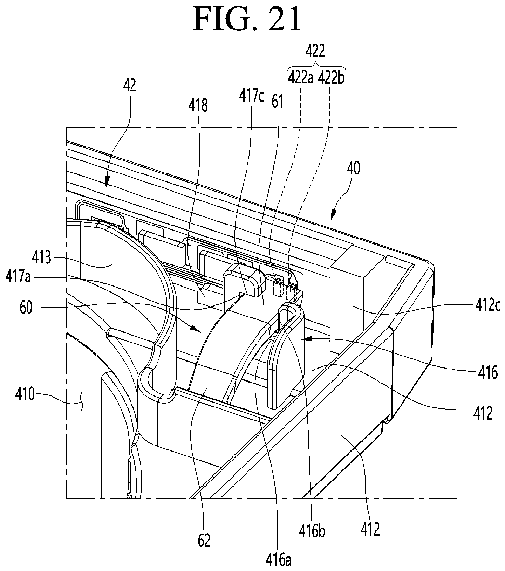

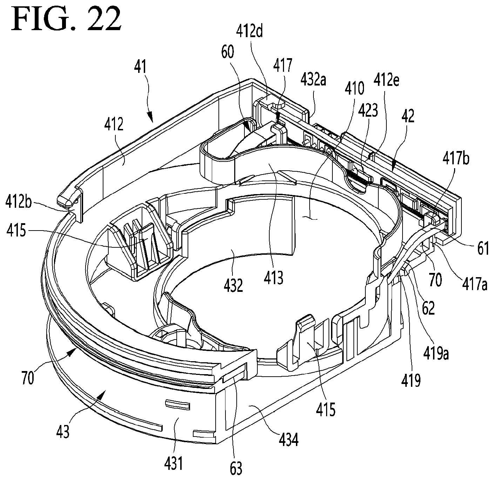

Fig. 20 is a plan view of the upper body that is one component of the lighting assembly, Fig. 21 is an enlarged view of a portion A of Fig. 15, and Fig. 22 is a cutaway perspective view taken along line XXII-XXII' of Fig. 15.

As illustrated in the drawings, the lighting module 42 may be mounted on the second half of the upper body 41. In addition, the light guide 60 may be mounted on the upper body 41, and the rear end of the light guide 60 may be disposed very close to the LED 422 of the lighting module 42.

Looking at this in more detail, the light substrate 421 is formed in the upper body 41 by the side fixing portions 412c and 412d, the substrate support 412e, and the substrate restriction portion 418 formed on the upper body 41 may be fixedly mounted.

The side fixing portions 412c and 412d may be disposed on an upper edge 412 defining the rear surface of the upper body 41. In addition, the side fixing portions 412c and 412d protrude from positions corresponding to the left and right both ends of the light substrate 421 to fix the light substrate 421 on both left and right sides, and left-right flow and up-down flow may be restricted. That is, one side fixing portion 412c of the side fixing portions 412c and 412d provided on the left and right sides restricts the side end of the light substrate 421, and the other side fixing portion 412d may restrict the side end and the top of 421.

In addition, the substrate support 412e protruding forward may be further disposed on the upper edge 412. The substrate support 412e may be formed at a position corresponding to the central portion of the light substrate 421 and may protrude up to the rear surface of the light substrate 421 to support the light substrate 421 from the rear.

The upper base 411 may be further provided with a substrate restriction portion 418 protruding to the top. A plurality of the substrate restriction portions 418 may be disposed along the lower end of the light substrate 421 and restrict the lower front end of the light substrate 421 to prevent the light substrate 421 from moving in the front-rear direction or being separated. In this manner, the position of the light substrate 421 may be maintained while being mounted in the upper body.

In the light guide 60, the round portion 63 may be disposed along the support 414, and a mounting position may be maintained by the support 414 and the diffusion member 70. In addition, the connection portion 62 may pass through the through holes 419 disposed on both sides of the upper base 411 to extend inside the upper body 41.

The through-hole 419 may be disposed behind the barrier 413, that is, inside the space partitioned by the barrier 413 and may be defined in each of both left and right sides of the upper opening 410. In addition, the through-hole 419 may be defined in the front along the same extension as the position of the LED 422.

Also, a guide seating portion 419a provided to be inclined or rounded may be further provided at one side and a rear side of the through-hole 419. In addition, the guide seating portion 419a may be formed to correspond to an inclination or a round shape of the connection portion 62. Therefore, the connection portion 62 may be maintained in a state of being seated on the guide seat portion 419a while passing through the through-hole 419, and the light guide 60 does not move and may be maintained in a stable mounting state.

The guide restriction portions 416 and 417 for restricting the extension portion 61 of the light guide 60 may be disposed between the through-hole 419 and the light substrate 421. The guide restriction portions 416 and 417 may protrude upward from the upper base 411 and may be disposed at a position corresponding to the arrangement position of the extension portion 61. Further, the guide restriction portions 416 and 417 may be respectively disposed in the same shape on both left and right sides to restrict the pair of extension portions 61, respectively.

The guide restriction portions 416 and 417 may include a first restriction portion 416 and a second restriction portion 417. The first restriction portion 416 and the second restriction portion 417 may be provided on both left and right sides of the extension portion 61, and may restrict the extension portion 61 from moving in the left and right directions and up and down directions.

In detail, the first restriction portion 416 may be disposed further outside (right side in Fig. 17) than the extension portion 61 and may extend upward to restrict one end of the extension portion 61. The first restriction portion 416 may include a first side restriction portion 416b that is in contact with one end of the extension portion 61 and a lower restriction portion 416a that is in contact with a lower surface of the extension portion 61. The first side restriction portion 416b and the lower restriction portion 416a may be connected to each other to have a stepped shape and may support one edge of the extension portion 61.

In addition, the second restriction portion 417 may be disposed further inside (left in Fig. 17) than the extension portion 61 and may extend upward to restrain the other end of the extension portion 61. The second restriction portion 417 may include a second side restriction portion 417a that is in contact with the other end of the extension portion 61 and an upper restriction portion 417b that is in contact with the top surface of the extension portion 61. The second side restriction portion 417a and the upper restriction portion 417b may be connected to each other to have a stepped shape, and may support the other edge of the extension portion 61.

Thus, when the extension portion 61 passes through the first restriction portion 416 and the second restriction portion 417, all the upper, lower, left, and right sides of the extension portion 61 may be restricted by the guide restriction portions 416 and 417. In addition, in the round portion 63 may be restricted in movement in the front and rear direction by the support 414 and the diffusion member 70, and a distance between the end of the extension portion 61 and the LED 422 may be maintained. For example, a gap between the rear end of the extension portion 61 and the front surface of the LED 422 may be maintained between about 0.5 mm and about 2 mm.

As a result, the light guide 60 may be mounted on the upper body 41 to be maintained without moving in the mounted state. In particularly, the end of the extension portion 61 may be maintained at an accurate mounting position by the guide restriction portions 416 and 417 and may be disposed in front of the LED 422.

In a state in which the light guide 60 is mounted in the accurate position, the rear end of the extension portion 61 may be disposed very close to the front surface of the LED 422. In addition, a plurality of the LEDs 422 may be disposed in the area between the left and right side ends of the rear end of the extension portion 61, and when the LED 422 is turned on, all the light emitted from the LED 422 may be directed to the extension portion 61 so that the ring light 730 is shined with sufficient brightness.

Hereinafter, the operation of the dispenser 20 according to an embodiment of the present invention having the above configuration will be described with reference to the drawings.

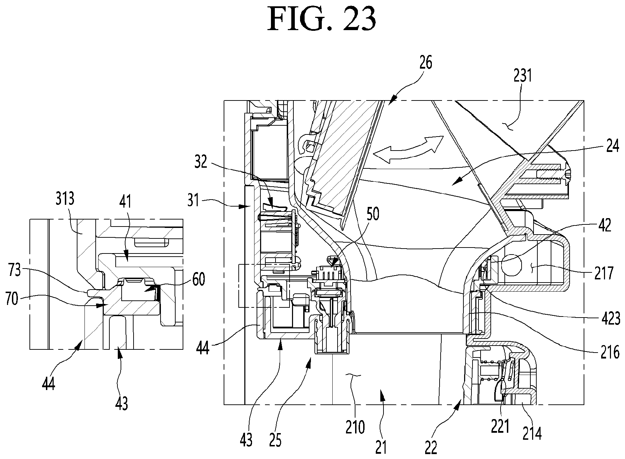

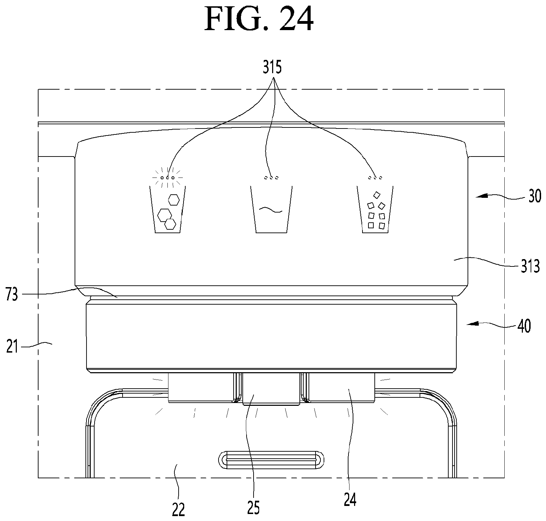

Fig. 23 is a cross-sectional view taken along line XXIII-XXIII' of Fig. 2, and Fig. 24 is a view illustrating a state of the dispensing portion when ice is dispensed through the dispenser.

As shown in the drawings, the user may set the state of the dispenser 20 by performing touch manipulation of the manipulation portion 313 of the touch assembly 30. That is, the user may set the operation state of the dispenser 20 by touch-manipulating any one of the plurality of touch portions 316 provided on the manipulation portion 313, and in the set operation state, the light irradiated form the corresponding light emitting member 331 may pass through the display 315, and thus, the user may recognize the light. For example, the user may select ice cubes, cube ice, and purified water through the manipulation portion 313, and the display 315 may be lit above the selected menu.

In a state in which the dispenser 20 is set to the manipulated ice or cube ice discharging mode by the user's manipulation of the manipulation portion 313, when the user operates the lever 22 to input the ice dispensing manipulation, as illustrated in Fig. 19. The shutter 26 may be opened. Then, the ice made in the ice maker 12 moves through the connection passage 23, passes through the ice chute 24, and may be discharged downward. After the operation of the lever 22 is finished or the set ice is discharged, the lever 22 is rotated to block the opening of the connection passage 23.

In addition, before the operation of the lever 22 for the operation of taking out the ice is performed, the state of the dispensing portion 27 is the ice chute 24 as illustrated in Fig. 3 due to the LED 422 being turned off may be in a state in which light is not emitted.

Also, when the lever 22 is operated to dispense the ice, the chute LED 423 may be turned on. When the suit LED 423 is turned on, the suit LED 423 irradiates light to the lower portion of the ice suit 24. Also, the light irradiated from the chute LED 423 may illuminate the ice chute 24, and in particular, the lower end of the ice chute 24 exposed below the lighting module 42 may emit light and be visualized to the outside.

Thus, the user may intuitively know that ice is being dispensed of the ice chute 24 through the brightly illuminated lower end of the ice chute 24, which is the outlet from which the ice is dispensed.

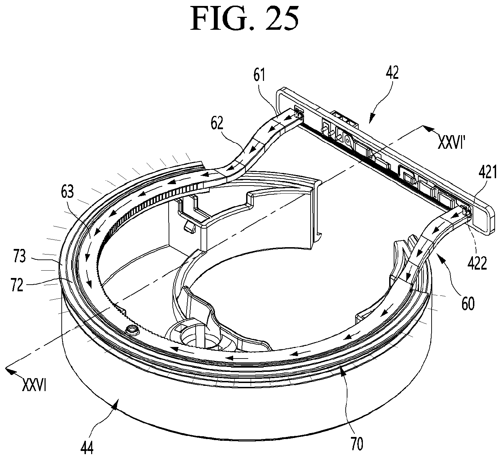

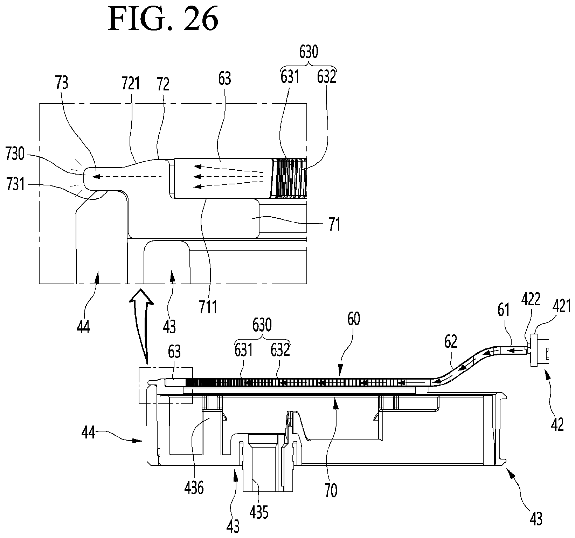

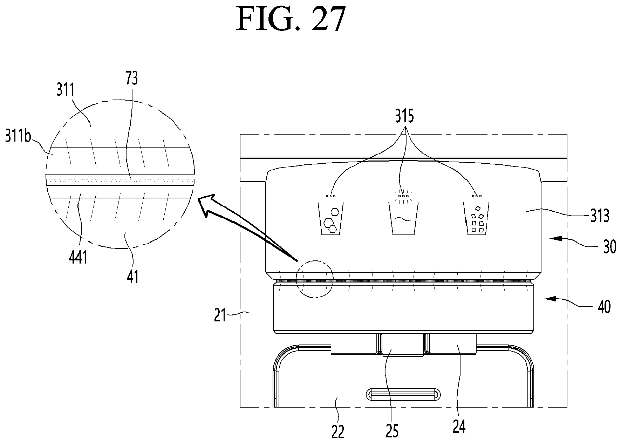

Fig. 25 is a perspective view illustrating an assembled state of the light guide and the diffusion member, Fig. 26 is a cross-sectional view taken along line XXVI-XXVI' of Fig. 21, and Fig. 27 is a view illustrating a state of the dispensing portion when water is dispensed through the dispenser.

When the dispenser 20 is set to the purified water discharging mode by the user's manipulation of the manipulation portion 313, and the user operates the lever 22 to input the purified water dispensing operation, a valve (not shown) may be opened so that water is supplied to the nozzle assembly 50 through the pipe 501, and the purified water may be discharged through the water outlet 25.

Before the operation of the lever 22 for dispensing purified water is performed, the state of the dispensing portion 27 becomes in a state in which the LED 422 is turned off, and thus, as illustrated in Fig. 3, the ring light 730 does not emit light.

Also, when the lever 22 operates to dispense the purified water, water is dispensed through the nozzle 55, and the LED 422 is turned on at the same time. When the LED 422 is turned on, the light of the LED 422 is irradiated to the extension portion s 61 on each of both sides of the light guide 60, and the light may pass through the connection portion 62 and then transmitted to the round portion 63. Then, the light directed to the round portion 63 may be reflected by the pattern portion 630 and may be directed to the outside including the front side. At this time, light is irradiated to the outside from the entire area of the round portion 63 due to the shape of the pattern portion 630.

Also, the light irradiated to the outside from the round portion 63 is diffused while passing through the diffusion member 70 that is in contact with the round portion 63, and shined with uniform brightness throughout the diffusion member 70. Thus, the exposed portion 73 exposed to the outside, i.e., the ring light 730 is shined in a ring shape of uniform brightness.

When the purified water is dispensed, any one of the plurality of LEDs 422 may operate. For example, when the extraction operation of the purified water is input, a white LED 422b among the plurality of LEDs 422 is turned on, and the exposed portion 73 exposed through the water outlet 25 as illustrated in Fig. 23., that is, the ring light 730 is shined in white.

Thus, the user may intuitively confirm that purified water is dispensed of the dispenser 20.

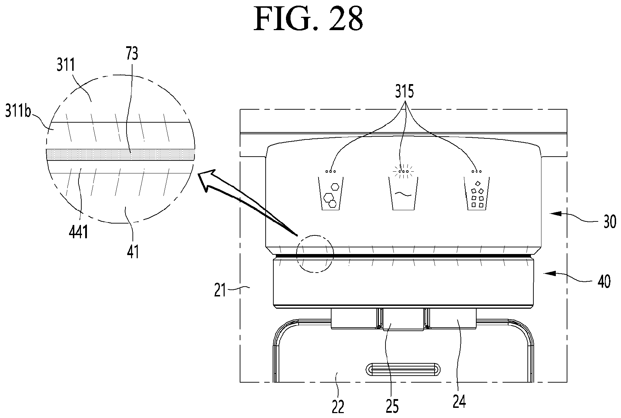

Fig. 28 is a view illustrating a state of the dispensing portion when a nozzle of the dispenser is sterilized.

The sterilization device 52 of the nozzle assembly 50 performs a sterilization operation of sterilizing by irradiating ultraviolet rays into the inside of the nozzle 55 according to a set logic. The sterilization operation may be performed every set time without a separate operation. For example, the sterilization LED 522 may be configured to be heated for about 10 minutes every about 50 minutes, and the inside of the nozzle 55 may be sterilized by the operation of the sterilization LED 522. Of course, the operation period and operation time of the sterilization LED 522 may be adjustable according to the user's operation setting.

In addition, the sterilization operation may be performed immediately by inputting operation start at a user's desired moment. Here, the user may directly input the sterilization operation by manipulating the manipulation portion 313 or by manipulating a separate manipulation member or display provided in the door 10, cabinet, or storage space other than the manipulation portion 313. Also, if necessary, it will be possible to directly input the sterilization operation using a remote device using the user's mobile phone, personal computer, or remote terminal.

When the sterilization operation starts, the LED 422 may be turned on. When the LED 422 is turned on, the light irradiated from the LED 422 may pass through the light guide 60 and be emitted through the diffusion member 70. Thus, the entire exposed portion 73 exposed to the outside, that is, the ring light 730, is illuminated in a ring shape of uniform brightness.

Also, when the sterilization operation starts, any one of the plurality of LEDs 422 may operate, and in order to distinguish the operation of the LED from the operation in which the purified water is dispensed, the diffusion member 70 that is, the ring light 730 may operate to be shined in a color different from the color when the purified water is dispensed.

For example, when the start signal of the sterilization operation is input, the blue LED 422a among the plurality of LEDs 422 is turned on, and as illustrated in Fig. 24, the diffusion member 70, that is, the ring light 730 in the water outlet 25 illuminates in blue.

Thus, the user may intuitively confirm that the sterilization operation of the nozzle 55 is performed by the dispenser 20.

In addition to the foregoing embodiment, various embodiments may be exemplified.

According to another embodiment, the ring light 730 is configured as a single ride guide. In another embodiment, other components except for the light guide 60 are the same as in the above-described embodiment, and thus, the same components are denoted by the same reference numerals, and detailed descriptions thereof will be omitted.

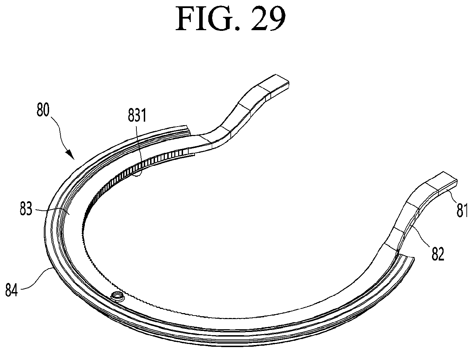

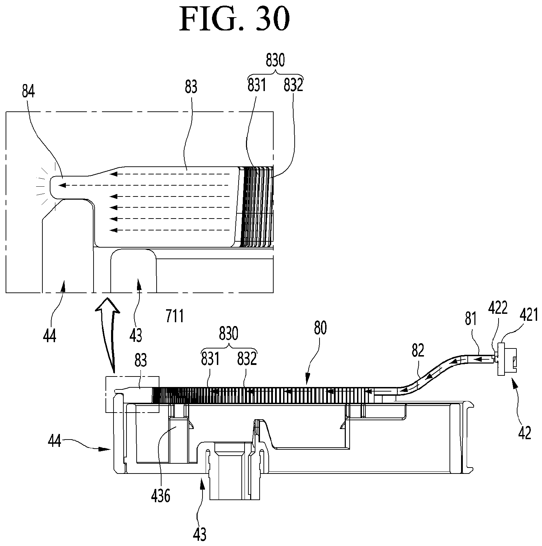

Fig. 29 is a perspective view of a light guide according to another embodiment, and Fig. 30 is a cross-sectional view illustrating a state in which the light guide is assembled.

According to another embodiment, the light guide 80 may be disposed between the upper body 41 and the lower body 43 constituting the dispenser 20, and the light guide 80. An exposed portion 84 may be exposed between the upper body 41 and the lower body 43.

The light guide 80 may include an extension portion 81 extending from the front of the LED 422, and a connection portion 82 extending downward from an end of the extended portion 81 and connected to both ends of the round portion 83 and a round portion 83 disposed along the circumference of the upper body 41.

In addition, a pattern portion 830 having an unevenness shape may be disposed on an inner surface of the round portion 83. The pattern portion 830 may have a structure of continuous grooves 831 and protrusions 832 and may be provided in the same manner as in the above-described embodiments.

In addition, an exposed portion 84 protruding forward may be disposed on the front surface of the round portion 83. The exposed portion 84 may be disposed at a position facing the pattern area 830 and may be disposed between the upper body 41 and the lower body 43 to be exposed to the outside. Thus, the exposed portion 84 may be shined in a ring shape when the LED 422 is turned on and may be referred to as a ring light.

In a state in which the light guide 80 is mounted, a lower surface of the light guide 80 may be supported by an upper end of the lower body 43, that is, an upper end of the lower edge 431. In addition, a lower surface of the exposed portion 84 may be supported by an upper end of the lower cover 44.

When the LED 422 is turned on while the lighting module 42 is mounted, the light irradiated from the LED 422 moves along the light guide 80. Also, the light moving along the light guide 80 is reflected to the outside including the front in the pattern portion 830, is radiated to the outside through the exposed portion 84, and shines in a ring shape so that it may be visualized by the user.

In another embodiment, except that an unlocking display 320 is added to the manipulation portion 313, other components are the same as in the above-described embodiment, so the same components are indicated using the same reference numerals and detailed descriptions thereof Is to be omitted

Hereinafter, a dispenser according to another embodiment and a refrigerator including the same will be described.

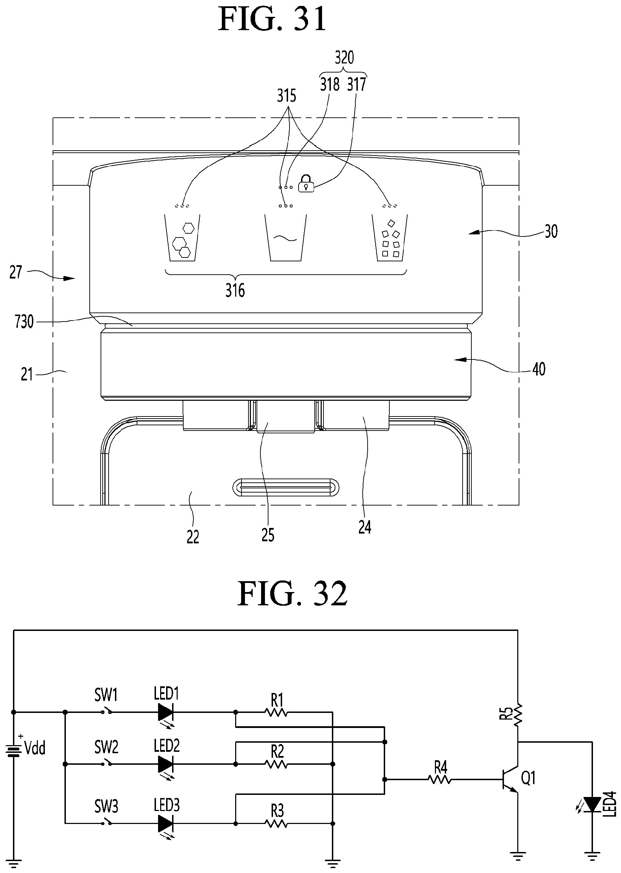

Fig. 31 is a front view of a dispenser according to another embodiment, and Fig. 32 is a perspective view illustrating a driving circuit of the dispenser according to another embodiment.

According to another embodiment, the unlocking display 320 may be further provided on the touch assembly 30 constituting the dispenser 20. In the unlocking display 320, for example, when the cube-state ice-purified water and the crushed ice do not operate due to the input of the manipulation portion 313 or the lever 22, the unlocking display 320 may be turned on so that the user is capable of recognizing the unlocked state.

The unlocking display 320 may include a guide 317 indicating the unlocking by a figure or a symbol, and a light emitting portion 318 through which light irradiated through a light emitting member or the like is transmitted. The unlocking display 320 may be disposed on the top of the touch assembly 30. Since cube-shaped ice, purified water, or pulverized ice is not dispensed at the same time from the unlocking display 320, the respective displays 315 indicating its operation state are not turned on at the same time. Also, when the unlocking display 320 is not in an operation state for dispensing ice or purified water, the unlocking display 320 may be turned on.

The touch assembly 30 according to an embodiment is provided in a rounded curved surface shape, and the touch substrate 33 has a relatively small size, so that the unlocking of the lock in addition to the driving circuit necessary to operate the necessary ice or water. There may be insufficient space to add a circuit for operating the display 320.

According to another embodiment, in the touch assembly 30, since there is no situation in which each of the displays 315 that display the operation state of the cube ice, water, or crushed ice is turned on at the same time, when all of the displays 315 are turned off, a circuit in which the LED of the display 320 operates may be provided.

When described in detail with reference to the drawings, SW1, SW12, and SW13 simplify the driving circuit of cube ice, purified water, and crushed ice with a switch, and LED1, LED 12, and LED 13 are three LEDs of cube ice, purified water, and crushed ice, respectively. Also, LED4 indicates an unlocked state.

When any one of SW1, SW2, and SW3 driving circuit operates, current is supplied to the base of Q1 through R4 and Q1 is turned on. Here, LED4 is connected to GND through Q1's Collector-Emitter and does not light up. That is, the unlocking display 320 is not turned on.

When all driving circuits of SW1, SW2, and SW3 do not operate, current is not supplied to the base of Q1, so LED4 is connected through VDD and R5 and turns on. That is, the unlocking display 320 may be turned on.

As such an operation circuit, an operation circuit for performing the operation of the unlocking display 320 on the touch substrate 33 formed in a relatively small size may be included.

In addition, there is an advantage in that the user may visually inform whether the user is in a state in which the user's input may be received through the manipulation portion 313 or the lever 22 through the unlocking display 320.

The dispenser and the refrigerator provided with the dispenser according to the proposed embodiment may have the following effects.

According to an embodiment, the operation state of the dispenser may be displayed through the ring light disposed along the circumference of the dispensing portion, and thus, the operation state of the dispenser may be displayed without the separate additional display to improve the assemblability and productivity of the dispenser.

Particularly, since the structure that emits light like the ring light is capable of being provided through the simple structures such as the light guide, and the pair of LEDs, and the substrate, the configuration may be simplified, and the productivity may be improved.

In addition, the space in which the ring light that irradiates light may be minimized, and also, only the ring light portion may be exposed to the outside, and the remaining components may be disposed inside the dispensing portion to realize the compact and neat structure of the dispenser.

In addition, the sterilizer that sterilizes the nozzle may be provided in the dispenser, and even when the sterilizer operates, the operation may be displayed externally through the emission of the ring light without adding the separate display.

Therefore, the operation state of the sterilizer that is provided inside the dispenser may be visualized to the outside to give the operation reliability to the user, and also, the sterilization operation of the nozzle may be intuitively indicated through the operation of the ring light in the dispensing portion adjacent to the nozzle so that the user recognizes the sterilization operation.

In addition, since whether the sterilizer is in operation is visualized, the user may check the normal operation of the sterilization operation of the nozzle, and in case of the abnormal operation, the abnormal operation may be immediately checked to ensure the operation and the sterilizer and the sterilization operation.

In addition, the LED that illuminates the ring light may be provided in plurality having the different colors, and thus, the ring light may emit light in different colors according to the dispensing of purified water and the sterilization operation so that the operation state is distinguished through the color of the ring light. Thus, the user may check the intuitively use and the operation state of the dispenser.

The light guide that illuminates the ring light may have the structure in which the pattern portion is disposed so that the entire ring light is brightly illuminated by irradiating the light in the outward direction to reflect the light to the outside, and the uniform brightness may be secured through the arrangement of the pattern portion.

In addition, the diffusion member may be disposed at the outside of the light guide so that the light irradiated from the light guide is diffused. Therefore, the ring light may be illuminated with the uniform brightness as a whole like the surface illumination, and the shadow of the pattern portion may be prevented from being visible to the outside.

In the dispenser, the touch assembly may be provided above the dispensing portion, and the touch device may be disposed to be in close contact with the rear surface of the touch assembly having the rounded curve shape so that the user is capable of easily recognizing the touch manipulation.

In addition, the display that displays the set state of the dispenser may be further provided on the front surface of the touch assembly together with the touch portion that induces the user's touch manipulation. In this case, the display may be provided by partially removing the blocking layer disposed on the front surface of the touch case made of the transparent material.

Thus, it is possible to realize the same effect as the fine hole through which the light irradiated from the light emitting member of the touch assembly is transmitted without the direct transmission, and also, the display including the fine openings having the uniform size may be molded.

According to an embodiment, the touch portion disposed on the front surface of the touch assembly to induce the user's touch operation may detect the user's operation through the capacitive touch sensor. In addition, the transfer member that detects the user's touch may be completely in close contact with the rear surface of the manipulation portion provided in the curved shape by the elastic member, and thus, even if the user manipulates the touch portion disposed on the manipulation portion, the effective touch manipulation may be detected.

## Claims

1. A lighting assembly for a dispenser of a refrigerator, comprising:
a dispensing body portion (45) including an upper body (41) and a lower body (43) coupled to each other, the dispensing body portion (45) having a cylindrical shape;
a light guide (60, 80) disposed between the upper body (41) and the lower body (43) and having a round portion (63, 83) with an arc shape;
a light diffusion member (70) disposed between the upper body (41) and the lower body (43) and having an arc shape surrounding an outer circumferential portion of the light guide (60); and
at least one LED (422) disposed to face one end of the light guide (60, 80),
wherein a circumferential outer portion of the light diffusion member (70) is exposed to an outside of the dispensing body portion (45) as an exposed portion (73) to emit light.

2. The lighting assembly according to claim 1, wherein the round portion (63, 83) of the light guide (60, 80) is exposed to an outside of the dispensing body portion (45) to emit light.

3. The lighting assembly according to claim 1, wherein the light diffusion member (70) comprises:
a diffusion member base (71) supporting the light guide (60); and
a front extension portion (72) protruding from the diffusion member base (71) at an outer circumferential portion thereof, the front extension portion (72) being in contact with the outer circumferential portion of the light guide (60).

4. The lighting assembly according to any one of the preceding claims, wherein a pattern portion (630, 830) is formed on an inner circumferential surface of the round portion (63, 83) to direct light towards the outside of the dispensing portion body (45), the pattern portion including a plurality of grooves (631, 831) extending in parallel.

5. The lighting assembly according to claim 4, wherein a width of the grooves (631, 831) of the pattern portion (630, 830) gradually increases from the ends of the round portion (63, 83) toward a center thereof and/or wherein an interval between adjacent grooves (631, 831) gradually decreases from the ends of the round portion (63, 83) toward the center thereof.

6. The lighting assembly according to any one of the preceding claims, wherein the light guide (60, 80) comprises an extension portion (61, 81) extending from the end of the round portion (63, 83) to face the LED (422).

7. The lighting assembly according to claim 6, wherein the light guide (60, 80) comprises a connection portion (62, 82) connecting the round portion (63, 83) and the extension portion (61, 81) and being inclined.

8. The lighting assembly according to claim 7, wherein the round portion (63, 83) is disposed on a bottom surface of the upper body (42) and the connection portion (62, 82) passes through a through-hole (419) defined in the bottom surface of the upper body (42), and
the LED (422) is provided on a light substrate (421) mounted in the upper body (42).

9. The lighting assembly according to any one of the preceding claims, wherein the dispensing portion body (45) includes at least one of a nozzle insertion portion (435) to accommodate a nozzle for dispensing water and/or a lower opening (433) to allow an ice chute (24) to pass through.

10. A refrigerator, comprising:
a cabinet (10) defining a storage space;
a door (11) configured to open and close the storage space;
a dispenser (20) provided on the door (11) and including at least one of an ice chute (24) defining a passage for dispensing ice and a nozzle (25) for dispensing water; and
a lighting assembly (40) according to any one of the preceding claims, the lighting assembly (40) surrounding the at least one of the ice chute (24) and the nozzle (25).

11. The refrigerator of claim 10, further comprising a touch assembly (30) for a user to operate the dispenser (20), wherein the touch assembly (30) comprises:
a manipulation portion (313) having a curved shape;
a first touch sensor (332a) and a second touch sensor (332b) installed on a touch substrate (33) having a flat plate shape, the touch sensors (332a, 332b) being covered by the manipulation portion (313);
a first elastic member (351a) installed between the first touch sensor (332a) and the manipulation portion (313) at a center of the manipulation portion (313), and
a second elastic member (352b) installed between the second touch sensor (332b) and the manipulation portion (313) at a periphery of the manipulation portion (313),
wherein the first elastic member and the second elastic member respectively have a surface in contact with the manipulation portion (313).

12. The refrigerator according to claim 11, wherein the touch assembly further comprises a guide member (34) provided between the manipulation portion (313) and the touch substrate (33),
wherein the guide member (34) has a surface in contact with the manipulation portion (313) that has a curved shape corresponding to the curved shape of the manipulation portion (313), and
wherein the guide member (34) has a guide hole (341) accommodating the first elastic member (351a) and the second elastic member (351b).

13. The refrigerator according to claim 11 or 12, wherein the touch assembly (30) further comprises:
a light emitting member (331) provided on the touch substrate (33);
a blocking layer (314) configured to block light emitted from the light emitting member (331), the blocking layer (314) being formed on the manipulation portion (313); and
a display (315) configured to transmit the light emitted from the light emitting member (331).

14. The refrigerator according to claim 13 when depending on claim 12, wherein the guide member (34) has a light hole (342) extending from the touch substrate (33) to the manipulation portion (313) to guide the light emitted from the light emitting member (331) toward the display (315).

## Patentansprüche

1. Beleuchtungsbaugruppe für einen Spender eines Kühlschranks, umfassend:
einen Ausgabekörperabschnitt (45), der einen oberen Körper (41) und einen unteren Körper (43) einschließt, die aneinandergekoppelt sind, wobei der Ausgabekörperabschnitt (45) eine zylindrische Form aufweist;
einen Lichtleiter (60, 80), der zwischen dem oberen Körper (41) und dem unteren Körper (43) angeordnet ist und einen runden Abschnitt (63, 83) mit einer Bogenform aufweist;
ein Lichtdiffusionselement (70), das zwischen dem oberen Körper (41) und dem unteren Körper (43) angeordnet ist und eine Bogenform aufweist, die einen äußeren Umfangsabschnitt des Lichtleiters (60) umgibt; und
mindestens eine LED (422), die dazu angeordnet ist, einem Ende des Lichtleiters (60, 80) zugewandt zu sein,
wobei ein äußerer Umfangsabschnitt des Lichtdiffusionselements (70) zu einer Außenseite des Ausgabekörperabschnitts (45) als ein freiliegender Abschnitt (73) freigelegt ist, um Licht zu emittieren.

2. Beleuchtungsbaugruppe nach Anspruch 1, wobei der runde Abschnitt (63, 83) des Lichtleiters (60, 80) zu einer Außenseite des Ausgabekörperabschnitts (45) freigelegt ist, um Licht zu emittieren.

3. Beleuchtungsbaugruppe nach Anspruch 1, wobei das Lichtdiffusionselement (70) Folgendes umfasst:
eine Diffusionselementbasis (71), die den Lichtleiter (60) stützt; und
einen vorderen Erstreckungsabschnitt (72), der von der Diffusionselementbasis (71) an einem äußeren Umfangsabschnitt davon hervorsteht, wobei der vordere Erstreckungsabschnitt (72) mit dem äußeren Umfangsabschnitt des Lichtleiters (60) in Kontakt steht.

4. Beleuchtungsbaugruppe nach einem der vorstehenden Ansprüche, wobei ein Musterabschnitt (630, 830) auf einer inneren Umfangsfläche des runden Abschnitts (63, 83) gebildet ist, um Licht zu der Außenseite des Ausgabeabschnittskörpers (45) hin zu führen, wobei der Musterabschnitt eine Vielzahl von Nuten (631, 831) einschließt, die sich parallel erstrecken.

5. Beleuchtungsbaugruppe nach Anspruch 4, wobei eine Breite der Nuten (631, 831) des Musterabschnitts (630, 830) von den Enden des runden Abschnitts (63, 83) zu einer Mitte davon hin allmählich zunimmt und/oder wobei ein Intervall zwischen angrenzenden Nuten (631, 831) von den Enden des runden Abschnitts (63, 83) zu der Mitte davon hin allmählich abnimmt.

6. Beleuchtungsbaugruppe nach einem der vorstehenden Ansprüche, wobei der Lichtleiter (60, 80) einen Erstreckungsabschnitt (61, 81) umfasst, der sich von dem Ende des runden Abschnitts (63, 83) erstreckt, um der LED (422) zugewandt zu sein.

7. Beleuchtungsbaugruppe nach Anspruch 6, wobei der Lichtleiter (60, 80) einen Verbindungsabschnitt (62, 82) umfasst, der den runden Abschnitt (63, 83) und den Erstreckungsabschnitt (61, 81) verbindet und geneigt ist.

8. Beleuchtungsbaugruppe nach Anspruch 7, wobei der runde Abschnitt (63, 83) auf einer unteren Fläche des oberen Körpers (42) angeordnet ist und der Verbindungsabschnitt (62, 82) durch ein Durchgangsloch (419) verläuft, das in der unteren Fläche des oberen Körpers (42) definiert ist, und
die LED (422) auf einem Lichtsubstrat (421) bereitgestellt ist, das in dem oberen Körper (42) montiert ist.

9. Beleuchtungsbaugruppe nach einem der vorstehenden Ansprüche, wobei der Ausgabeabschnittskörper (45) mindestens eines von einem Stutzeneinführabschnitt (435), um einen Stutzen zum Ausgeben von Wasser unterzubringen, und/oder einer unteren Öffnung (433), um zu ermöglichen, dass ein Eisschacht (24) dort hindurchverläuft, einschließt.

10. Kühlschrank, umfassend:
einen Schrank (10), der einen Aufbewahrungsraum definiert,
eine Tür (11), die dazu konfiguriert ist, den Aufbewahrungsraum zu öffnen und zu schließen;
einen Spender (20), der an der Tür (11) bereitgestellt ist und mindestens eines von einem Eisschacht (24), der einen Durchgang zum Ausgeben von Eis definiert, und/oder einem Stutzen (25) zum Ausgeben von Wasser einschließt; und
eine Beleuchtungsbaugruppe (40) nach einem der vorstehenden Ansprüche, wobei die Beleuchtungsbaugruppe (40) das mindestens eine von dem Eisschacht (24) und dem Stutzen (25) umgibt.

11. Kühlschrank nach Anspruch 10, weiter umfassend eine Berührungsbaugruppe (30) für einen Benutzer, um den Spender (20) zu bedienen, wobei die Berührungsbaugruppe (30) Folgendes umfasst:
einen Betätigungsabschnitt (313), der eine gekrümmte Form aufweist;
einen ersten Berührungssensor (332a) und einen zweiten Berührungssensor (332b), die auf einem Berührungssubstrat (33) installiert sind, das eine flache Plattenform aufweist, wobei die Berührungssensoren (332a, 332b) durch den Betätigungsabschnitt (313) bedeckt sind;
ein erstes elastisches Element (351a), das zwischen dem ersten Berührungssensor (332a) und dem Betätigungsabschnitt (313) in einer Mitte des Betätigungsabschnitts (313) installiert ist, und
ein zweites elastisches Element (352b), das zwischen dem zweiten Berührungssensor (332b) und dem Betätigungsabschnitt (313) an einer Peripherie des Betätigungsabschnitts (313) installiert ist,
wobei das erste elastische Element und das zweite elastische Element jeweils eine Fläche in Kontakt mit dem Betätigungsabschnitt (313) aufweisen.

12. Kühlschrank nach Anspruch 11, wobei die Berührungsbaugruppe weiter ein Leitungselement (34) umfasst, das zwischen dem Betätigungsabschnitt (313) und dem Berührungssubstrat (33) bereitgestellt ist,
wobei das Leitungselement (34) eine Fläche in Kontakt mit dem Betätigungsabschnitt (313) aufweist, die eine gekrümmte Form aufweist, die der gekrümmten Form des Betätigungsabschnitts (313) entspricht, und
wobei das Leitungselement (34) ein Leitungsloch (341) aufweist, das das erste elastische Element (351a) und das zweite elastische Element (351b) unterbringt.

13. Kühlschrank nach Anspruch 11 oder 12, wobei die Berührungsbaugruppe (30) weiter Folgendes umfasst:
ein lichtemittierendes Element (331), das auf dem Berührungssubstrat (33) bereitgestellt ist;
eine Blockierschicht (314), die dazu konfiguriert ist, von dem lichtemittierenden Element (331) emittiertes Licht zu blockieren, wobei die Blockierschicht (314) auf dem Betätigungsabschnitt (313) gebildet ist; und
eine Anzeige (315), die dazu konfiguriert ist, von dem lichtemittierenden Element (331) emittiertes Licht durchzulassen.

14. Kühlschrank nach Anspruch 13, wenn abhängig von Anspruch 12, wobei das Leitungselement (34) ein Lichtloch (342) aufweist, das sich von dem Berührungssubstrat (33) zu dem Betätigungsabschnitt (313) erstreckt, um das von dem lichtemittierenden Element (331) emittierte Licht zu der Anzeige (315) hin zu leiten.

## Revendications

1. Ensemble d'éclairage pour un distributeur d'un réfrigérateur, comportant :
une partie de corps de distribution (45) incluant un corps supérieur (41) et un corps inférieur (43) couplés l'un à l'autre, la partie de corps de distribution (45) ayant une forme cylindrique ;
un conduit lumineux (60, 80) disposé entre le corps supérieur (41) et le corps inférieur (43) et ayant une partie ronde (63, 83) en forme d'arc ;
un élément de diffusion de lumière (70) disposé entre le corps supérieur (41) et le corps inférieur (43) et ayant une forme d'arc entourant une partie circonférentielle extérieure du conduit lumineux (60) ; et
au moins une LED (422) disposée de manière à faire face à une extrémité du conduit lumineux (60, 80),
dans lequel une partie circonférentielle extérieure de l'élément de diffusion de lumière (70) est exposée à un extérieur de la partie de corps de distribution (45) comme une partie exposée (73) pour émettre de la lumière.

2. Ensemble d'éclairage selon la revendication 1, dans lequel la partie ronde (63, 83) du conduit lumineux (60, 80) est exposée à un extérieur de la partie de corps de distribution (45) pour émettre de la lumière.

3. Ensemble d'éclairage selon la revendication 1, dans lequel l'élément de diffusion de lumière (70) comporte :
une base d'élément de diffusion (71) supportant le conduit lumineux (60) ; et
une partie d'extension avant (72) faisant saillie à partir de la base d'élément de diffusion (71) sur une partie circonférentielle extérieure de celle-ci, la partie d'extension avant (72) étant en contact avec la partie circonférentielle extérieure du conduit lumineux (60).

4. Ensemble d'éclairage selon l'une quelconque des revendications précédentes, dans lequel une partie de motif (630, 830) est formée sur une surface circonférentielle intérieure de la partie ronde (63, 83) pour diriger la lumière vers l'extérieur du corps de partie de distribution (45), la partie de motif incluant une pluralité de rainures (631, 831) s'étendant de manière parallèle.

5. Ensemble d'éclairage selon la revendication 4, dans lequel une largeur des rainures (631, 831) de la partie de motif (630, 830) augmente graduellement à partir des extrémités de la partie ronde (63, 83) vers un centre de celle-ci et/ou dans lequel un intervalle entre des rainures (631, 831) adjacentes diminue graduellement à partir des extrémités de la partie ronde (63, 83) vers le centre de celle-ci.

6. Ensemble d'éclairage selon l'une quelconque des revendications précédentes, dans lequel le conduit lumineux (60, 80) comporte une partie d'extension (61, 81) s'étendant à partir de l'extrémité de la partie ronde (63, 83) pour faire face à la LED (422).

7. Ensemble d'éclairage selon la revendication 6, dans lequel le conduit lumineux (60, 80) comporte une partie de liaison (62, 82) reliant la partie ronde (63, 83) et la partie d'extension (61, 81) et étant inclinée.

8. Ensemble d'éclairage selon la revendication 7, dans lequel la partie ronde (63, 83) est disposée sur une surface inférieure du corps supérieur (42) et la partie de liaison (62, 82) passe à travers un trou traversant (419) défini dans la surface inférieure du corps supérieur (42), et
la LED (422) est agencée sur un substrat lumineux (421) monté dans le corps supérieur (42).

9. Ensemble d'éclairage selon l'une quelconque des revendications précédentes, dans lequel le corps de partie de distribution (45) inclut au moins un élément parmi une partie d'insertion de buse (435) destinée à recevoir une buse pour distribuer de l'eau et/ou une ouverture inférieure (433) pour permettre à une glissière à glaçons (24) de passer à travers.

10. Réfrigérateur, comportant :
une armoire (10) définissant un espace de stockage ;
une porte (11) configurée pour ouvrir et fermer l'espace de stockage ;
un distributeur (20) agencé sur la porte (11) et incluant au moins un élément parmi une glissière à glaçons (24) définissant un passage de distribution de glaçons et une buse (25) pour distribuer de l'eau ; et
un ensemble d'éclairage (40) selon l'une quelconque des revendications précédentes, l'ensemble d'éclairage (40) entourant le au moins un élément parmi la glissière à glaçons (24) et la buse (25).

11. Réfrigérateur selon la revendication 10, comportant en outre un ensemble tactile (30) pour qu'un utilisateur actionne le distributeur (20), dans lequel l'ensemble tactile (30) comporte :
une partie de manipulation (313) ayant une forme incurvée ;
un premier capteur tactile (332a) et un second capteur tactile (332b) installés sur un substrat tactile (33) ayant une forme de plaque plane, les capteurs tactiles (332a, 332b) étant recouverts par la partie de manipulation (313) ;
un premier élément élastique (351a) installé entre le premier capteur tactile (332a) et la partie de manipulation (313) sur un centre de la partie de manipulation (313), et
un second élément élastique (352b) installé entre le second capteur tactile (332b) et la partie de manipulation (313) sur une périphérie de la partie de manipulation (313),
dans lequel le premier élément élastique et le second élément élastique ont respectivement une surface en contact avec la partie de manipulation (313).

12. Réfrigérateur selon la revendication 11, dans lequel l'ensemble tactile comporte en outre un élément de guidage (34) agencé entre la partie de manipulation (313) et le substrat tactile (33),
dans lequel l'élément de guidage (34) a une surface en contact avec la partie de manipulation (313) qui a une forme incurvée correspondant à la forme incurvée de la partie de manipulation (313), et
dans lequel l'élément de guidage (34) a un trou de guidage (341) recevant le premier élément élastique (351a) et le second élément élastique (351b).

13. Réfrigérateur selon la revendication 11 ou 12, dans lequel l'ensemble tactile (30) comporte en outre :
un élément électroluminescent (331) agencé sur le substrat tactile (33) ;
une couche de blocage (314) configurée pour bloquer la lumière émise à partir de l'élément électroluminescent (331), la couche de blocage (314) étant formée sur la partie de manipulation (313) ; et
un affichage (315) configuré pour transmettre la lumière émise à partir de l'élément électroluminescent (331).

14. Réfrigérateur selon la revendication 13 lorsque dépendante de la revendication 12, dans lequel l'élément de guidage (34) a un trou lumineux (342) s'étendant du substrat tactile (33) à la partie de manipulation (313) pour guider la lumière émise à partir de l'élément électroluminescent (331) vers l'affichage (315).
